# EUROPEAN PATENT SPECIFICATION

(11) **EP 4 251 102 B1**
(45) Date of publication and mention of the grant of the patent: **03.06.2026**
(21) Application number: 21835015.5
(22) Date of filing: 29.11.2021
(51) Int. Cl.: A61F 5/44, A61F 5/455

(54) **FLUID COLLECTION DEVICES WITH ANATOMICAL FIT CROSS-REFERENCE TO RELATED APPLICATIONS**
FLÜSSIGKEITSSAMMELVORRICHTUNGEN MIT ANATOMISCHER PASSFORM QUERVERWEIS AUF VERWANDTE ANWENDUNGEN
DISPOSITIFS DE COLLECTE DE FLUIDES AVEC AJUSTEMENT ANATOMIQUE RÉFÉRENCE À DES APPLICATIONS CONNEXES

(30) Priority: 30.11.2020 US 202063119161 P
(43) Date of publication of application: 04.10.2023
(73) Proprietor: Purewick Corporation, Covington, Georgia 30014-1498 (US)
(72) Inventor: JOHANNES, Ashley Marie, Statham, Georgia 30066 (US); WHITTOME, Samuel Edmund, Cambridge CB1 9GQ (GB); WIN, Leanne Yip Heung, Middlesex HA98RH (GB); MITCHELL, Martyn, Royston Hertfordshire (GB)
(74) Representative: Studio Torta S.p.A.
(86) International application number: PCT/US2021/060993
(87) International publication number: WO 2022/115692

(56) References cited:
- WO-A1-2019/212951
- DE-U1- 202015 104 597
- US-A1- 2018 228 642

## Description

### BACKGROUND

An individual may have limited or impaired mobility such that typical urination processes are challenging or impossible. For example, the individual may have surgery or a disability that impairs mobility. In another example, the individual may have restricted travel conditions such as those experience by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters be may be uncomfortable, painful, and may cause urinary tract infections.

Thus, users and manufacturers of fluid collection devices continue to seek new and improved devices, systems, and methods to collect urine. Examples of such devices, systems, and methods are disclosed in WO2019212951A1.

### SUMMARY

The invention is set out in the appended set of claims.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1A** is an isometric view of a fluid collection device, according to an embodiment.
**FIG. 1B** is a cross-sectional view of the fluid collection device of FIG. 1A along the plane A-A.
**FIG. 1C** is an end view of the fluid collection device of **FIG. 1A** along the longitudinal axis B-B.
**FIG. 2A** is an isometric view of a fluid collection device, according to an embodiment.
**FIG. 2B** is a side cross-sectional view of the fluid collection device of **FIG. 2A****,** taken along the plane **D-D.**
**FIG. 2C** is a cross-sectional view of the fluid collection device of **FIG. 2A** along the plane E-E.
**FIG. 2D** is an end view of the fluid collection device of **FIG. 2A** along the axis C-C.
**FIG. 3A** is an isometric view of a fluid collection device, according to an embodiment.
**FIG. 3B** is a close-up view of the window F in **FIG. 3A****.**
**FIG. 4A** is an isometric view of a fluid collection device, according to an embodiment.
**FIG. 4B** is a close-up view of the window G in **FIG. 4A****.**
**FIG. 5A** is an exploded isometric view of a fluid collection device, according to an embodiment.
**FIG. 5B** is a side view of the fluid collection device of **FIG. 5A** in a deployed state, according to an embodiment.
**FIG. 6A** is an exploded isometric view of a fluid collection device, according to an embodiment.
**FIG. 6B** is a side view of the fluid collection device of **FIG. 6A** in a deployed state, according to an embodiment.
**FIG. 7A** is an isometric view of fluid collection device, according to an embodiment.
**FIG. 7B** is an end view of the fluid collection device of **FIG. 7A** taken along the longitudinal axis H-H.
**FIG. 8A** is an isometric view of the fluid collection device, according to an embodiment.
**FIG. 8B** is an end view of the fluid collection device of **FIG. 8A** viewed along the longitudinal axis J-J.
**FIG. 9** is a block diagram of a system for fluid collection, according to an embodiment.
**FIG. 10** **is** a flow diagram of a method to collect fluid, according to an embodiment.

### DETAILED DESCRIPTION

Embodiments disclosed herein are related to devices, systems, and methods for collecting fluids from an individual. The fluid collection devices, systems, and methods disclosed herein include a fluid collection devices having a means for retaining the fluid collection device in a position for use between the legs of a wearer. The means for retaining are shaped to provide a selected interface between the wearer and the device to prevent the device from being shifted, dislodged, or otherwise misaligned with the wearers urethra during use.

The fluid collection devices herein include a fluid impermeable barrier that at least partially defines a chamber. The fluid impermeable barrier also defines an opening extending therethrough that is sized, shaped, and located to be positioned adjacent to a urethra. The fluid collection devices includes a porous material at least partially disposed within the chamber to receive urine from the urethra of a wearer. The fluid collection devices include a means for retaining the fluid collection device in a position for use between the legs of a wearer. The fluid(s) collected by the fluid collection devices may include at least one of urine, vaginal discharge, blood, sweat, or other bodily fluids. The fluid collection devices may also include a conduit disposed in the chamber to remove the fluid(s) therefrom.

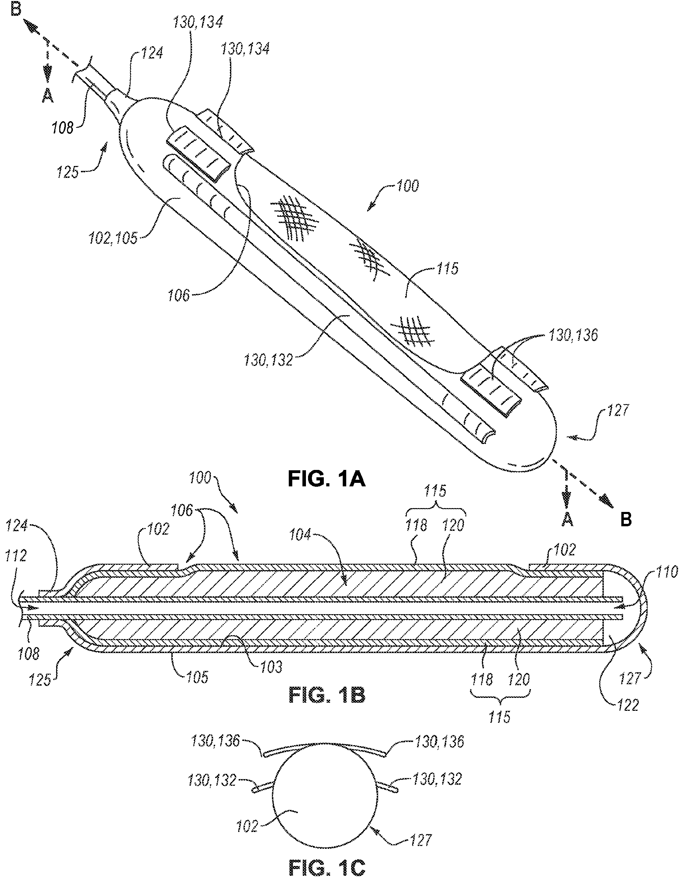

**FIG. 1A** is an isometric view of a fluid collection device 100, according to an embodiment. **FIG. 1B** is a cross-sectional view of the fluid collection device 100 of **FIG. 1** along the plane A-A. **FIG. 1C** is an end view of the fluid collection device 100 of **FIG. 1A** along the longitudinal axis B-B. The fluid collection device 100 includes a fluid impermeable barrier 102 defining a chamber 104 therein, porous material 115 at least partially disposed in the chamber 104, and an optional conduit 108 at least partially disposed within the chamber 104. The fluid collection device 100 includes means for retaining 130 the fluid collection device in a position for use between the legs of a wearer.

The fluid impermeable barrier 102 at least partially defines the chamber 104 *(e.g.,* interior region) and an opening 106. For example, the inner surface(s) 103 of the fluid impermeable barrier 102 at least partially defines the chamber 104 within the fluid collection device 100. The fluid impermeable barrier 102 at least temporarily retains the fluid(s) in the chamber 104. The fluid impermeable barrier 102 may be formed of any suitable fluid impermeable material(s), such as a fluid impermeable polymer (*e.g*., silicone, polypropylene, polyethylene, polyethylene terephthalate, thermoplastic elastomer(s), a polycarbonate, etc.), a metal film, natural rubber, another suitable material, or combinations thereof. As such, the fluid impermeable barrier 102 substantially prevents the fluid(s) from passing therethrough.

In an example, the fluid impermeable barrier 102 may be air permeable and liquid impermeable. In such an example, the fluid impermeable barrier 102 may be formed of a hydrophobic material that defines a plurality of pores that are air permeable but not liquid permeable. In an example, one or more portions of at least an outer surface of the fluid impermeable barrier 102 may be formed from a soft and/or smooth material, thereby reducing chaffing.

In some examples, the fluid impermeable barrier 102 may be tubular (ignoring the opening), such as substantially cylindrical (as shown), oblong, prismatic, flattened tube, or any other extruded shape (*e.g.,* a tube having multiple lobes separated by one or more webs therebetween, such as an I-beam shape or the like). The fluid impermeable barrier 102 may be sized to fit between the legs of a wearer. During use, an outer surface 105 of the fluid impermeable barrier 102 may at least partially contact the wearer.

The opening 106 provides an ingress route for fluids to enter the chamber 104. The opening 106 may be defined by the fluid impermeable barrier 102, such as by an inner edge of the fluid impermeable barrier 102. For example, the opening 106 is formed in and extends through the fluid impermeable barrier 102, from the outer surface 105 to the inner surface 103, thereby enabling fluid(s) to enter the chamber 104 from outside of the fluid collection device 100. The opening 106 may be located and shaped to be positioned adjacent to a wearer's urethra while the device is in use. At least a portion of porous material(s) disposed in the chamber 104 may be exposed through the opening 106 to allow fluids to move inwardly into the chamber 104, such as via one or more of permeation, suction, or wicking.

The fluid collection device 100 may be positioned proximate to the urethra and urine may enter the chamber 104 via the opening 106. When in use, the opening 106 may be elongated, extending from a first location below the urethra to a second location above the urethra *(e.g.,* at or near the top of the vaginal opening or the pubic region). The opening 106 may exhibit an elongated shape because the space between the legs of a wearer is relatively narrow when the legs of the wearer are closed, thereby only permitting the flow of the fluid(s) along a path that corresponds to the elongated shape of the opening 106 (*e.g*., longitudinally extending opening). The opening 106 in the fluid impermeable barrier 102 may exhibit a length that is measured along the longitudinal axis of the fluid collection device 100 that may be at least about 10% of the length of the fluid collection device 100, such as about 25% to about 50%, about 40% to about 60%, about 50% to about 75%, about 65% to about 85%, or about 75% to about 95% of the length of the fluid collection device 100.

The opening 106 in the fluid impermeable barrier 102 may exhibit a width that is measured transverse to the longitudinal axis of the fluid collection device 100 and may be at least about 10% of the circumference of the fluid collection device 100, such as about 25% to about 50%, about 40% to about 60%, about 50% to about 75%, about 65% to about 85%, or about 75% to about 100% of the circumference of the fluid collection device 100. The opening 106 may exhibit a width that is greater than 50% of the circumference of the fluid collection device 100 since the vacuum (e.g., suction) through the conduit 108 pulls the fluid through the porous material 115 and into the conduit 108. The opening 106 may be longitudinally oriented *(e.g.,* having a major axis parallel to the longitudinal axis of the device 100). In some examples (not shown), the opening 106 may be laterally oriented (*e.g.,* having a major axis perpendicular to the longitudinal axis of the device 100).

The means for retaining 130 may be disposed on or at least partially defined by the fluid impermeable barrier 102. For example, the means for retaining 130 may include one or more flanges 132, 134, and 136. The one or more flanges 132, 134, and 136 may be disposed on *(e.g.,* attached to or integrally formed with) the fluid impermeable barrier 102. The one or more flanges 132, 134, and 136 may include flanges extending outwardly from the fluid impermeable barrier 102 a selected distance, such as at least 0.2 cm from the outer surface 105 of the fluid impermeable barrier 102, 0.2 cm to 2 cm, 0.2 cm to 1 cm, 1 cm to 2 cm, less than 2 cm, less than 1.5 cm, or less than 1 cm. The one or more flanges 132, 134, and 136 may have a thickness of at least 1 mm, such as 1 mm to 8 mm, 1 mm to 3 mm, 3 mm to 6 mm, 5 mm to 8 mm, less than 1 cm, or less than 5 mm.

The one or more flanges 132, 134, and 136 may be formed of the same material as the fluid impermeable barrier 102, such as silicone, HDPE, LDPE, latex, or the like. The one or more flanges 132, 134, and 136 may be semi-rigid, malleable, or deformable responsive to pressure applied thereto. For example, the one or more flanges 132, 134, and 136 may have a composition and dimensions that provide at least partially flexible (e.g., resiliently flexible) flanges.

The one or more flanges 132, 134, and 136 may extend perpendicularly, obliquely, or tangentially from the outer surface 105. For example, the flange 132 may extend substantially perpendicularly or obliquely from the fluid impermeable barrier 102 about half way up the outer surface 105 (when the opening in positioned on top of the fluid collection device 100) and the flanges 134 and 136 may extend substantially tangentially or obliquely from the top of the fluid impermeable barrier 102. In a non-deformed state, the one or more flanges 132, 134, and 136 may be linear when viewed in cross-section along the line B-B. In a non-deformed state, the one or more flanges 132, 134, and 136 may be at least partially arcuate when viewed in cross-section along the line B-B. The one or more flanges 132, 134, and 136 may be deflected downward when placed in position between the legs of a wearer. Accordingly, the one or more flanges 132, 134, and 136 may then resist bending back toward the conformation of the relaxed state by providing compressive forces against the wearer when the fluid impermeable barrier 102 or any other portion of the fluid collection device 100 is subjected to forces which would dislodge the fluid collection device 100. Accordingly, the one or more flanges 132, 134, and 136 may help retain the fluid collection device 100 in position during use.

The one or more flanges 132, 134, and 136 may extend longitudinally along at least a portion of the fluid impermeable barrier 102. For example, the flange(s) 132 may extend longitudinally along the fluid impermeable barrier 102 from the first end region 125 to the second end region 127. As shown in **FIGS. 1A** and **1C****,** the flange 132 may be positioned in a middle portion (e.g., midway between the bottom and the top of the fluid impermeable barrier) of the fluid impermeable barrier when viewed along the line B-B. The flanges 132 may extend outward from the fluid impermeable barrier 102 on both sides of the opening 106. For example, the flange(s) 132 may extend along the middle of the fluid impermeable barrier 102 below the opening 106 form the first end region 125 to the second end region 127.

The flange(s) 134 may extend from the first end region 125 toward the opening 106, such as on the top of the fluid impermeable barrier (*e.g*., when the opening 106 is oriented upward). The flange(s) 134 may extend outwardly away from (*e.g.,* tangentially or obliquely away from the longitudinal axis of) the top of the fluid impermeable barrier 102 in opposite directions.

The flange(s) 136 may extend from the second end region 127 toward the opening 106, such as on the top of the fluid impermeable barrier (*e.g.,* when the opening 106 is oriented upward). The flange(s) 136 may extend outwardly away from (*e.g.,* tangentially or obliquely away from the longitudinal axis of) the top of the fluid impermeable barrier 102 in opposite directions.

While flanges 132, 134, and 136 are depicted as extending from the top and middle portions of the fluid impermeable barrier, more or fewer sets of flanges may be included in some examples. In some examples (not shown), additional or replacement flanges may extend from any portion of the outer surface 105 of the fluid impermeable barrier, such as the bottom or additional medial portions.

The fluid collection device 100 includes the porous material 115 disposed in the chamber 104. The porous material 115 may extend across at least a portion (*e.g.,* all) of the opening 106. At least a portion of the porous material 115 may be exposed to an environment outside of the chamber 104 through the opening 106. The porous material 115 may be configured to wick any fluid away from the opening 106, thereby preventing the fluid from escaping the chamber 104. The permeable properties of the porous material referred to herein may be wicking, capillary action, diffusion, or other similar properties or processes, and are referred to herein as "permeable" and/or "wicking." Such "wicking" may not include absorption of fluid into the porous material. Put another way, substantially no absorption of fluid into the porous material may take place after the material is exposed to the fluid and removed from the fluid for a time. While no absorption is desired, the term "substantially no absorption" may allow for nominal amounts of absorption of fluid into the porous material (e.g., absorbency), such as less than about 10 wt% of the dry weight of the porous material, less than about 7 wt%, less than about 5 wt%, less than about 3 wt%, less than about 2 wt%, less than about 1 wt%, or less than about 0.5 wt% of the dry weight of the porous material. The porous material 115 may also wick the fluid generally towards an interior of the chamber 104.

The porous material 115 may include one or more of a fluid permeable membrane 118 or a fluid permeable support 120. The fluid permeable membrane 118 may include any porous material or a material that may wick the fluid. For example, the fluid permeable membrane 118 may include fabric, such as a gauze (*e.g.,* a silk, linen, or cotton gauze), another soft fabric, or another smooth fabric. The fluid permeable membrane 118 may include spun plastic fibers (*e.g.,* nylon), such as a spun plastic mat or bed. Forming the fluid permeable membrane 118 from gauze, soft fabric, and/or smooth fabric may reduce chaffing caused by the fluid collection device 100.

The fluid collection device 100 may include the fluid permeable membrane 118 disposed in the chamber 104. The fluid permeable membrane 118 may cover at least a portion (*e.g.,* all) of the opening 106. The fluid permeable membrane 118 may be composed to wick any fluid inwardly away from the opening 106, thereby preventing the fluid from escaping the chamber 104.

The porous material 115 of the fluid collection device 100 may include the fluid permeable support 120 disposed in the chamber 104. The fluid permeable support 120 is composed to support the fluid permeable membrane 118 since the fluid permeable membrane 118 may be formed from a foldable, flimsy, or otherwise easily deformable material. For example, the fluid permeable support 120 may be positioned such that the fluid permeable membrane 118 is disposed between the fluid permeable support 120 and the fluid impermeable barrier 102. As such, the fluid permeable support 120 may support and maintain the position of the fluid permeable membrane 118 thereon. The fluid permeable support 120 may include any material that may wick the fluid, such as any of the fluid permeable membrane materials disclosed herein. For example, the fluid permeable support 120 may be formed from any fluid porous material that is less deformable than the fluid permeable membrane 118, such as any of the materials disclosed herein for the fluid permeable membrane 118, in a more dense or rigid form. In some examples, the fluid permeable support 120 may include a porous polymer (e.g., nylon, polyester, polyurethane, polyethylene, polypropylene, etc.) structure, an open cell foam, or spun plastic fibers (*e.g*., nylon fibers). In some examples, the fluid permeable membrane 118 may include gauze and the fluid permeable support may include spun nylon fibers. In some examples, the fluid permeable support 120 may be formed from fabric, felt, gauze, or combinations thereof. In some examples, the fluid permeable support 120 may be formed from a natural material, such as cotton, wool, silk, or combinations thereof. In such examples, the material may have a coating to prevent or limit absorption of fluid into the material, such as a water repellent coating. In some examples, the fluid permeable support 120 may be omitted from the fluid collection device 100. In some examples, the fluid permeable membrane 118 may be optional. For example, the porous material 115 may include only the fluid permeable support 120.

The fluid permeable support 120 may have a greater permeability or a greater ability to wick fluids than the fluid permeable membrane 118, such as to move the fluid inwardly from the outer surface of the fluid collection device 100. In some examples, the permeability or the wicking ability of the fluid permeable support 120 and the fluid permeable membrane 118 may be substantially the same.

The fluid permeable membrane 118 and the fluid permeable support 120 may at least substantially completely fill the portions of the chamber 104 that are not occupied by the conduit 108. In another example, the fluid permeable membrane 118 and the fluid permeable support 120 may not substantially completely fill the portions of the chamber 104 that are not occupied by the conduit 108. In such an example, the fluid collection device 100 includes a reservoir 122 (FIG. 1B) in the chamber 104.

The fluid collection device 100 may include the conduit 108, which extends into the chamber 104. As illustrated in **FIG. 1B****,** the conduit 108 may be at least partially disposed in the chamber 104. The conduit 108 *(e.g.,* a tube) includes an inlet 110 at a first end region and an outlet 112 at a second end region positioned downstream from the inlet 110. The conduit 108 may extend into the chamber 104 to any point therein. For example, the conduit 108 may be inserted into the chamber at the first end region 125 of the fluid collection device 100 and extend therethrough to the second end region 127 of the fluid collection device 100. The conduit 108 may extend into the fluid impermeable barrier 102 from the first end region *125 (e.g.,* proximate to the outlet 112) through to the second end region 127 *(e.g.,* opposite the first end region 125) to a point proximate to the reservoir 122 such that the inlet 110 is in fluid communication with the reservoir 122. In some examples (not shown), the conduit 108 may enter the chamber 104 in the second end region 127 and the inlet 110 of the conduit 108 may be disposed in the second end region 127 *(e.g.,* in the reservoir 122 or flush with fluid impermeable barrier 102). The fluid collected in the reservoir 122 may be removed from the chamber 104 via the conduit 108. In some examples, the inlet 110 may be disposed at the end of the fluid permeable support 120 in the second end region 127, such as flush with the end of the fluid permeable support 120. In some examples, the inlet 110 may be disposed within the fluid permeable support 120 such between first end region 125 and the second end region 127.

The conduit 108 may include a flexible material such as plastic tubing *(e.g.,* medical tubing). Such plastic tubing may include a thermoplastic elastomer, polyvinyl chloride, ethylene vinyl acetate, polytetrafluoroethylene, etc., tubing. In some examples, the conduit 108 may include silicon or latex. In some examples, the conduit 108 may include one or more portions that are resilient, such as to by having one or more of a diameter or wall thickness that allows the conduit to be flexible. In some examples, the conduit 108 may be frosted or opaque *(e.g.,* black) to obscure visibility of the fluid(s) therein.

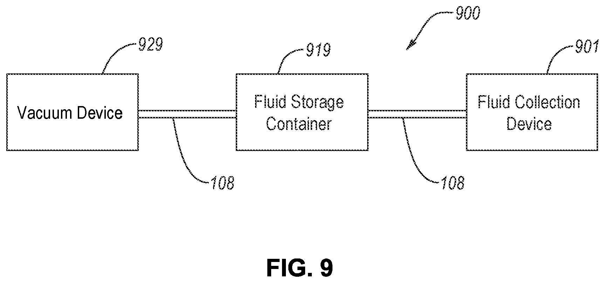

The fluid collection device 100 may be operably coupled to a vacuum source. For example, the conduit 108 fluidly couples an interior region of the chamber 104 with the fluid storage container **(****FIG. 9****)** or the vacuum source **(****FIG. 9****).** Accordingly, fluids may be removed from the chamber 104 via the conduit 108.

The fluid impermeable barrier 102, the fluid permeable membrane 118 and the fluid permeable support 120 may be sized and shaped to have the conduit 108 at least partially disposed in the chamber 104. For example, at least one of the fluid permeable membrane 118 and the fluid permeable support 120 may be configured to form a space that accommodates the conduit 108. The fluid impermeable barrier 102 may define an aperture 124 sized to receive the conduit 108. The at least one conduit 108 may be disposed in the chamber 104 via the aperture 124. The aperture 124 may be sized and shaped to form an at least substantially fluid tight seal against the conduit 108 thereby substantially preventing the fluid(s) from escaping the chamber 104. The fluid collected in the fluid collection device 100 may be removed from the chamber 104 via the conduit 108.

The porous material 115 i*(e.g.,* fluid permeable membrane 118 and the fluid permeable support 120) may not substantially completely fill the portions of the chamber 104 that are not occupied by the conduit 108. The fluid collection device 100 may include the reservoir 122 therein. As shown, the reservoir 122 is a substantially unoccupied portion of the chamber 104. The reservoir 122 may be defined between the fluid impermeable barrier 102 and the porous material 115 (*e.g.,* one or both of the fluid permeable membrane 118 and the fluid permeable support 120). The fluid(s) emitted by the wearer may be wicked into the chamber 104 by the porous material 115 and may flow through the fluid permeable membrane 118 and/or fluid permeable support 120 to the reservoir 122. The fluid impermeable barrier 102 may retain the fluid(s) in the reservoir 122. The reservoir 122 may be located in a portion of the fluid collection device expected to be positioned in a gravimetrically low point of the fluid collection device when worn by a user. While depicted in the second end region 127, the reservoir 122 may be located in any portion of the chamber 104 such as the first end region 125.

In some examples, the fluid permeable support 120 is spaced from at least a portion of the conduit 108 and the reservoir 122 may be defined between the fluid permeable support 120 and the conduit 108.

As shown in **FIG. 1B****,** the end of the conduit 108 may extend beyond the fluid permeable membrane 118 and/or fluid permeable support 120, such as into the reservoir 122. In some examples, the inlet 110 may not extend into the reservoir 122. In such examples, the inlet 110 may be disposed within the porous material 115 (fluid permeable membrane 118 and/or fluid permeable support 120) or at a terminal end thereof. For example, an end of the conduit 108 may be coextensive with or recessed within the fluid permeable membrane 118 and/or fluid permeable support 120.

Locating the inlet 110 at or near a location expected to be the gravimetrically low point of the chamber 104 when worn by a user enables the conduit 108 to receive more of the fluid(s) than if inlet 110 was located elsewhere and reduce the likelihood of pooling (*e.g.,* pooling of the fluid(s) may cause microbe growth and foul odors). For instance, the fluid(s) in the fluid permeable membrane 118 and the fluid permeable support 120 may flow in any direction due to capillary forces. However, the fluid(s) may exhibit a preference to flow in the direction of gravity, especially when at least a portion of the fluid permeable membrane 118 and/or the fluid permeable support 120 is saturated with the fluid(s). Accordingly, one or more of the inlet 110 or the reservoir 122 may be located in the second end region 127.

Other embodiments of fluid impermeable barriers, fluid permeable membranes, fluid permeable supports, chambers, conduits and their shapes and configurations are disclosed in U.S. Patent Application No. 15/612,325 filed on June 2, 2017; U.S. Patent Application No. 15/260,103 filed on September 8, 2016; and U.S. Patent No. 10,226,376 filed on June 1, 2017.

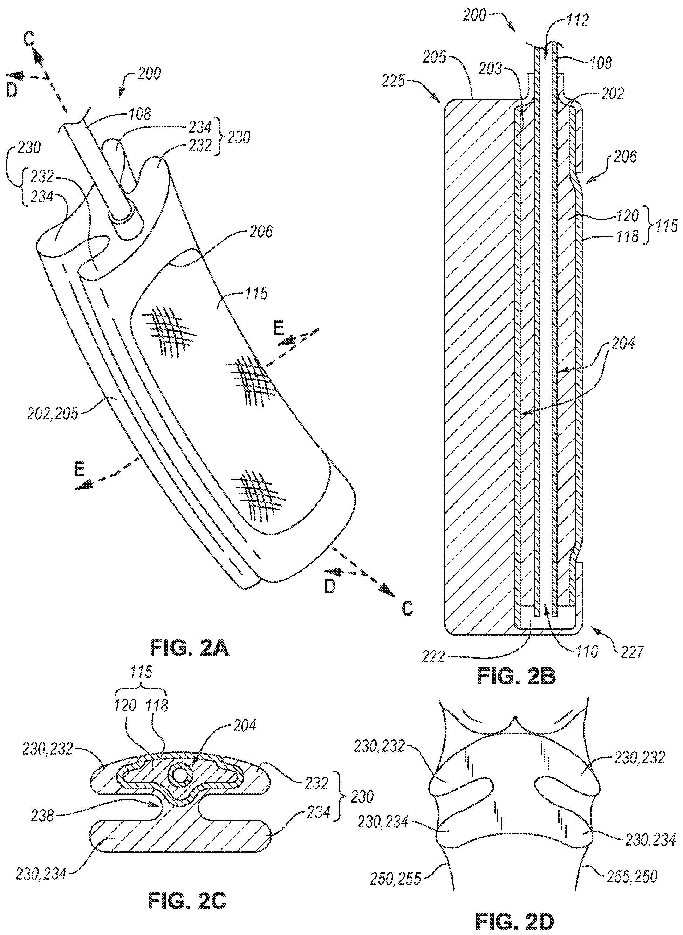

Means for retaining with various flange designs may be utilized to retain the fluid collection device in position on the wearer. For example, the shape of the fluid collection device, as a whole, may include a plurality of flange shaped lobes therein. **FIG. 2A** is an isometric view of a fluid collection device 200, according to an embodiment. **FIG. 2B** is a side cross-sectional view of the fluid collection device 200 of **FIG. 2A****.,** taken along the plane **D-D** **FIG. 2C** is a cross-sectional view of the fluid collection device 200 of **FIG. 2A** along the plane E-E, according to an embodiment. **FIG. 2D** is an end view of the fluid collection device 200 of **FIG. 2A** along the longitudinal axis C-C, according to an embodiment. The fluid collection device 200 includes a fluid impermeable barrier 202 defining a chamber 204 therein, porous material 115 at least partially disposed in the chamber 204, and an optional conduit 108 at least partially disposed within the chamber 204. The fluid collection device 200 includes means for retaining 230 the fluid collection device in a position for use between the legs of a wearer. The fluid impermeable barrier 202 may be similar or identical to the fluid impermeable barrier 102 in one or more aspects, such as material, position, thickness, or the like. For example, the fluid impermeable barrier 202 at least partially defines the chamber 204 (e.g., interior region) and an opening 206. The inner surface(s) 203 of the fluid impermeable barrier 202 at least partially define the chamber 204 within the fluid collection device 200. The fluid impermeable barrier 202 at least temporarily retains the fluid(s) in the chamber 204.

The fluid impermeable barrier 202 may be formed of any suitable fluid impermeable material(s), such as a fluid impermeable polymer (*e.g*., silicone, polypropylene, polyethylene, polyethylene terephthalate, thermoplastic elastomer(s), a polycarbonate, etc.), a metal film, natural rubber, another suitable material, or combinations thereof. As such, the fluid impermeable barrier 202 substantially prevents the fluid(s) from passing therethrough. The fluid impermeable barrier 202 may be at least partially malleable (*e.g.,* resiliently deformable) responsive to pressure applied thereto. Some portions of the fluid impermeable barrier 202 may be more malleable than other portions of the fluid impermeable barrier, such as in the flanges 232 and 234.

The means for retaining 230 are at least partially defined by the fluid impermeable barrier 202. For example, the means for retaining 230 include a plurality of flanges 232 and 234 in the body of the fluid collection device 200. The plurality of flanges 232 and 234 may be formed by one or more of the fluid impermeable barrier 202 or porous material 115.

The fluid collection device 200 may have the general shape of an I-beam with a plurality of flanges extending laterally away from a web extending therebetween. As shown in **FIGS. 2A** and **2C****,** the flanges 232 and 234 may be longitudinally extending lobes (along the longitudinally axis C-C) in the fluid impermeable barrier 202. For example, the flanges 232 and 234 may be formed by one or more of the fluid impermeable barrier 202 and the porous material 115. The plurality of flanges 232 and 234 may extend from the first end region 225 to the second end region 227 of the fluid collection device 200. The plurality of flanges 232 and 234 may extend laterally way from a web 238 therebetween by at least 1 cm, such as 1 cm to 10 cm, 1 cm to 3 cm, 3 cm to 6 cm, 6 cm to 10 cm, less than 6 cm, less than 4 cm, or less than 3 cm. The plurality of flanges 232 and 234 may be at least 3 mm thick, such as 3 mm to 2 cm, 3 mm to 7 mm, 6 mm to 1 cm, 1 cm to 1.5 cm, 1.5 cm to 2 cm, less than 2 cm, or less than 1.5 cm thick. As shown, the plurality of flanges may include two sets of longitudinally extending flanges 232 and 234 separated by the web 238 therebetween. More or fewer flanges may be used, such as one set of flanges, three sets of flanges, or more. In some examples, one or more of the plurality of flanges 232 or 234 may run along less than the entire longitudinal length of the fluid collection device 200. Such as only in the second end region 227.

Returning to **FIG. 2B****,** the fluid impermeable barrier 202 includes an outer surface 205 at least partially defining the outer shape of the fluid collection device 200 and an inner surface 203 at least partially defining the chamber 204 of the fluid collection device 200. The chamber 204 may be relatively wider than the chamber 104 **(****FIG. 1B****).** For example, the chamber 204 may be wider than the chamber 204 is deep. In some examples, the chamber 204 may parallel the shape of at least a portion of the outer surface 205. For example and as shown in **FIG. 2C****,** the chamber 204 may extend into at least a portion of the flanges 232 and into at least a portion of the web 238. In some examples (not shown), the flanges 232 and 234 may be formed by the porous material 115 and the fluid impermeable barrier 202, such as having a thin fluid impermeable barrier 202 retaining the porous material 215 in the lobes. In such examples, porous material 115 in the chamber 204 may form at least a portion of the flanges 232, 234 and the web 238.

The porous material 115 *(e.g.,* fluid permeable membrane 118 and fluid permeable support 120) may at least partially fill the chamber 204. The inner surface 203 may at least partially define a reservoir 222 in the chamber 204. The reservoir 222 may be defined between the inner surface 203, the porous material 115, and the conduit 108. The conduit 108 may be positioned in the fluid collection device 200 similarly or identically to the positioning with the fluid collection device 100.

As shown in **FIG. 2C****,** the flanges 232 and 234 may differ in material composition. For example, the flanges 232 may include a portion of the porous material 115 and the fluid impermeable barrier 202 disposed thereover, and the flanges 234 may only include the fluid impermeable barrier 202. In some examples, the flanges 234 may include a filler materials *(e.g.,* foam, silicone, or the like) disposed *(e.g.,* sealed) within the fluid impermeable barrier 202.

As shown in **FIG. 2D****,** the flanges 232 and 234 may at least partially conform to the shape of the wearer's 255 anatomy in contact therewith. For example, the flanges 232 and 234 may be bent downward responsive to forces applied thereto when positioning the fluid collection device 200 between the legs 255 of the wearer 250. In such examples, the relatively malleable flanges 232 and 234 may provide compression between the legs 255 and the fluid collection device 200 to retain the device in position adjacent to the urethra of the wearer 250. Additionally, the downward bend in the flanges 232 and 234 may resist bending in the opposite direction when dislodging forces are applied thereto, thereby preventing dislodgement.

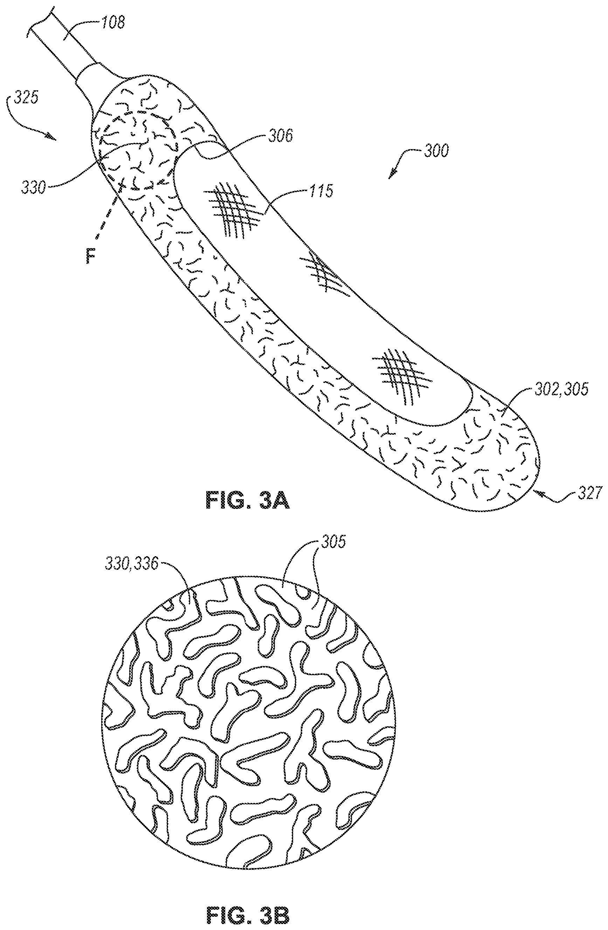

**In** some examples, the means for retaining may include a selected texturing on the fluid impermeable barrier. **FIG. 3A** is an isometric view of a fluid collection device 300, according to an embodiment. **FIG. 3B** is a close-up view of the window F in **FIG. 3A****.** The fluid collection device 300 includes the porous material 115, the conduit 108, and the fluid impermeable barrier 302 including the means for retaining 330. The fluid collection device 300 may be similar or identical to any of the fluid collection devices disclosed herein, in one or more aspects. The fluid impermeable barrier 302 may be similar or identical to the fluid impermeable barrier 102 **(FIGS. 1A-1D),** in one or more aspects (e.g., material composition, position, chamber, etc.). For example, the fluid impermeable barrier 302 includes an outer surface 305, an inner surface (not shown) defining a chamber therein, and an opening 306. The opening 306 allows the porous material 115 disposed at least partially within the chamber to be at least partially exposed to the environment outside of the chamber. The fluid collection device 300 includes a reservoir as disclosed herein with respect to the fluid collection device 100 **(****FIG. 1****).**

The means for retaining 330 includes texturing 336 formed on the outer surface 305 of the fluid impermeable barrier 302 or disposed over the outer surface 305 of the fluid impermeable barrier 302. For example, at least a portion of the outer surface 305 includes the texturing 336 extending outwardly therefrom. The texturing 336 may include randomly oriented raised bodies on the fluid impermeable barrier 302. The texturing 336 includes raised bodies that extend from the outer surface 305 a selected distance. The raised bodies of the texturing 336 define ridges 437 and valleys 438 on the outer surface 405, with the ridges 437 defining high points and the valleys 438 defining low-points of the outer surface 405. The ridges 437 of the texturing 336 may extend 1 mm or more from the valleys 438 on the outer surface 305, such as 1 mm to 15 mm, 1 mm to 5 mm, 5 mm to 10 mm, 10 mm to 15 mm, less than 15 mm, less than 10 mm, or less than 5 mm. The raised bodies of the texturing 336 may have a width of 1 mm or more, such as 1 mm to 10 mm, 1 mm to 3 mm, 3 mm to 6 mm, 6 mm to 10 mm, less than 10 mm, less than 5 mm, or less than 3 mm. The raised bodies of the texturing 336 may be spaced from each other by an average of 1 mm or more, such as 1 mm to 10 mm, 1 mm to 3 mm, 3 mm to 6 mm, 6 mm to 10 mm, less than 10 mm, less than 5 mm, less than 3 mm, at least 3 mm, or at least 5 mm. The ridges 437 and valleys 438 may make a portion of the fluid impermeable barrier having the texturing 336 more compressible than a portion of the fluid impermeable barrier 302 without the texturing 336 *(e.g.,* the portion of the fluid impermeable barrier 302 underlying the texturing).

The raised bodies may be integrally formed with (*e.g.,* molded) the rest of the fluid impermeable barrier 302 or may be adhered (*e.g.,* laminated) to the fluid impermeable barrier 302. In some examples, the raised bodies forming the texturing 336 are the same material as the rest of the fluid impermeable barrier 302 or may be a softer material than the fluid impermeable barrier 302. For example, the fluid impermeable barrier 302 may be formed of silicone and the raised features of the texturing 336 may be made of silicone, another material (*e.g.,* LDPE), or a softer silicone than the rest of the fluid impermeable barrier 302. By utilizing the softer material for texturing on the outer surface of the fluid collection device, sores may be prevented at the wearer-device interface.

The texturing 336 creates a larger surface area for interfacing with the wearer (e.g., skin of the wearer). Additionally, by utilizing raised bodies having a relatively thin width, the raised bodies allow the texturing 336 to provide a relatively compressible portion on the fluid impermeable barrier 302 to resist dislodging.

The texturing 336 may be disposed over all of the outer surface 305 of the fluid impermeable barrier 302. In some examples, only selected portions of the fluid impermeable barrier 302 may have texturing 336 thereon, such as only in the second end region 327 (which is expected to be placed in the gluteal cleft during use). Additionally or alternatively, the first end region 325 may include the texturing 336 thereon. The remaining (untextured) portions of the outer surface 305, such as a medial portion between the first end region 325 and the second end region 327, may be substantially smooth. The smooth portion(s) may prevent irritation of the skin of the wearer in contact therewith.

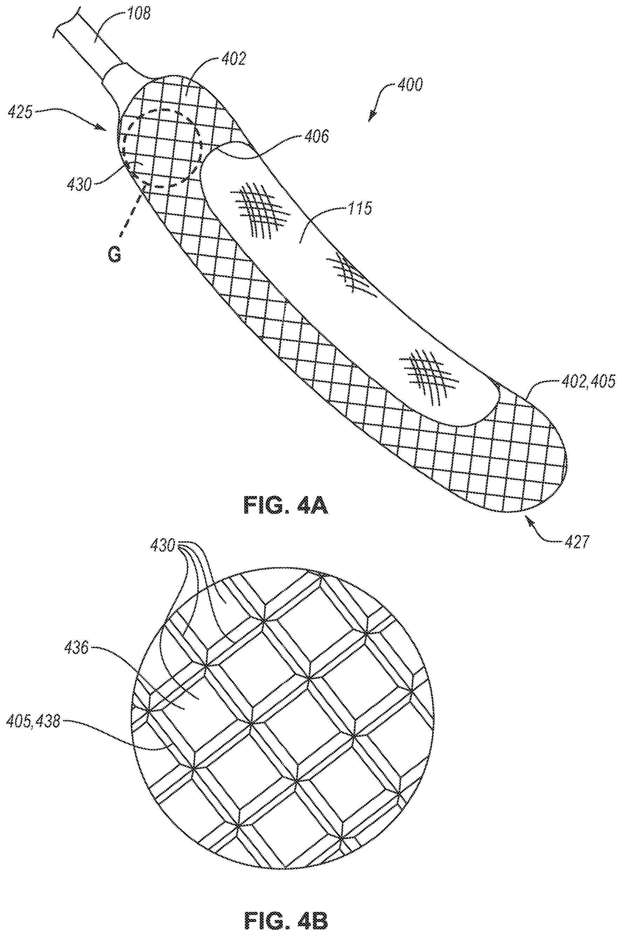

In some examples, the raised features forming the texturing may be disposed in a pattern. **FIG. 4A** is an isometric view of a fluid collection device 400, according to an embodiment. **FIG. 4B** is a close-up view of the window G in **FIG. 4A****.** The fluid collection device 400 includes the porous material 115, the conduit 108, and the fluid impermeable barrier 402 including the means for retaining 430. The fluid collection device 400 may be similar or identical to any of the fluid collection devices disclosed herein, in one or more aspects. The fluid impermeable barrier 402 may be similar or identical to the fluid impermeable barrier 102 **(FIGS. 1A-1D)** or 302 **(****FIGS. 3A-3B****),** in one or more aspects (e.g., material composition, position, chamber, etc.). For example, the fluid impermeable barrier 402 includes an outer surface 405, an inner surface (not shown) defining a chamber therein, and an opening 406. The opening 406 allows the porous material 115 disposed at least partially within the chamber to be at least partially exposed to the environment outside of the chamber. The fluid collection device 400 includes a reservoir as disclosed herein with respect to the fluid collection device 100 **(****FIG. 1****),** such as in the second end region 427.

The means for retaining 430 includes texturing 436 formed on the outer surface 405 of the fluid impermeable barrier 402 or disposed over the outer surface 405 of the fluid impermeable barrier 402. The texturing 436 includes raised bodies, that extend from the outer surface 405 a selected distance. For example, the texturing 436 includes a three dimensional pattern of raised bodies. The raised bodies may be sized and shaped to create a selected amount of friction or suction between the texturing 436 and a smooth surface (e.g., skin) when the smooth surface is in contact therewith. The raised bodies of the texturing 436 define ridges 437 and valleys 438 on the outer surface 405, with the ridges 437 defining high points and the valleys 438 defining low-points of the outer surface 405. The ridges 437 of the texturing 436 may extend 1 mm or more from the valleys 438, such as 1 mm to 15 mm, 1 mm to 5 mm, 5 mm to 10 mm, 10 mm to 15 mm, less than 15 mm, less than 10 mm, or less than 5 mm. The ridges 437 may have a width of 1 mm or more, such as 1 mm to 10 mm, 1 mm to 3 mm, 3 mm to 6 mm, 6 mm to 10 mm, less than 10 mm, less than 5 mm, or less than 3 mm. The ridges 437 may be spaced from each other by an average of 1 mm or more, such as 1 mm to 10 mm, 1 mm to 3 mm, 3 mm to 6 mm, 6 mm to 10 mm, less than 10 mm, less than 5 mm, less than 3 mm, at least 3 mm, or at least 5 mm. The ridges and valleys of the texturing 436 may make a portion of the fluid impermeable barrier 402 having the texturing 436 more compressible than a portion of the fluid impermeable barrier 402 without the texturing 436 (*e.g.,* the portion of the fluid impermeable barrier 402 underlying the texturing).

As shown, texturing 436 may include a three dimensional pattern, such as a repeating diamond pattern with a raised apex (*e.g.,* ridge) in a centroid of at least some diamonds in the repeating diamond pattern. While shown as a repeating diamond-shaped pattern, the texturing 436 may include a different pattern or shapes therein. Shapes of raised bodies may include circles, ovals, polygons, or any other shape. The raised bodies may taper from the ridge 437 to the valley 438, as shown. In some examples, the raised bodies may abruptly change from the ridge 437 to the valley 438 such as by a vertical surface therebetween. The raised bodies may have a flat upper surface. In some examples, the raised bodies may form suction cups.

The raised bodies forming the texturing 436 of the means for retaining 430 may be integrally formed with (*e.g*., molded) the rest of the fluid impermeable barrier 402 or may be adhered (*e.g*., laminated) to the fluid impermeable barrier 402. In some examples, the raised bodies forming the texturing 436 are the same material as the rest of the fluid impermeable barrier 402 or may be a softer material than the fluid impermeable barrier 402. For example, the fluid impermeable barrier 402 may be formed of silicone and the raised features of the texturing 436 may be made of silicone, another material (e.g., LDPE), or a softer silicone than the rest of the fluid impermeable barrier 402. The softer material of the texturing may prevent sores at the wearer-device interface.

The texturing 436 creates a larger surface area for interfacing with the wearer (e.g., skin of the wearer). Additionally, by utilizing raised bodies in the texturing 436, a selected amount of surface area or suction may be created between the fluid collection device 400 and the wearer's skin to resist dislodging.

The texturing 436 may be disposed over all of the outer surface 405 of the fluid impermeable barrier 402. In some examples, only selected portions of the fluid impermeable barrier 402 may have texturing 436 thereon, such as only in the second end region 427 (which is expected to be placed in the gluteal cleft during use). Additionally or alternatively, the first end region 425 may include the texturing 436 thereon. The remaining (untextured) portions of the outer surface 405, such as a medial portion between the first end region 425 and the second end region 427, may be substantially smooth. The smooth portion(s) may prevent irritation of the skin of the wearer in contact therewith.

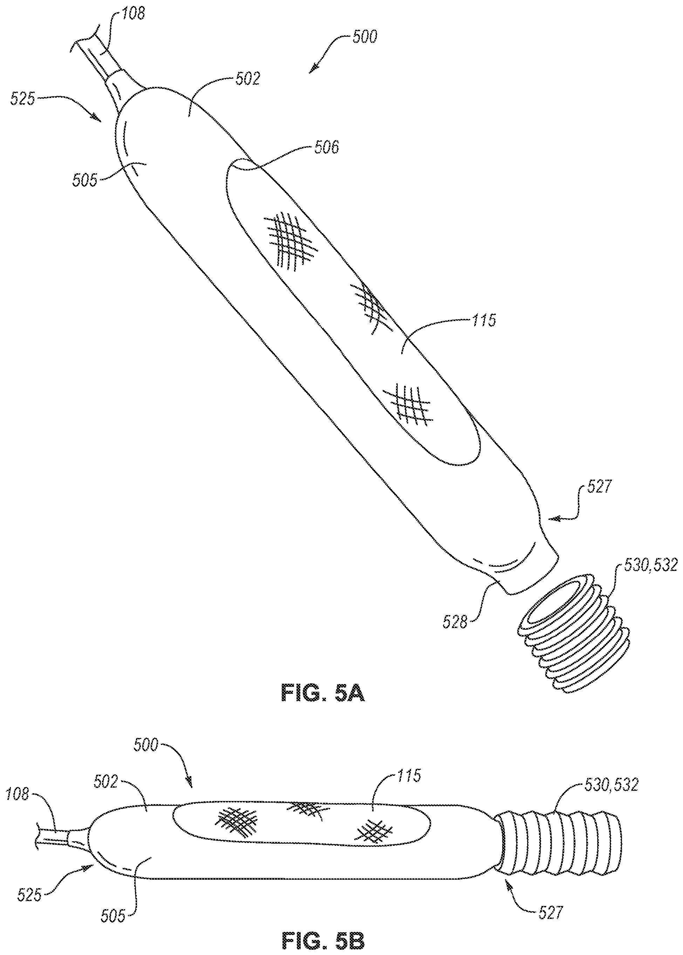

In some examples, the means for retaining may include an attachment that is attached to the fluid impermeable barrier. **FIG. 5A** is an exploded isometric view of a fluid collection device 500, according to an embodiment. **FIG. 5B** is a side view of the fluid collection device 500 of **FIG. 5A** in a deployed state, according to an embodiment. The fluid collection device 500 includes the porous material 115, the conduit 108, the fluid impermeable barrier 502, and the means for retaining 530. The fluid collection device 500 may be similar or identical to any of the fluid collection devices disclosed herein, in one or more aspects. The fluid impermeable barrier 502 may be similar or identical to the fluid impermeable barrier 102 **(FIGS. 1A-1D),** 302 **(****FIG. 3A****) or** 402 **(****FIG. 4A****),** in one or more aspects (e.g., material composition, position, chamber, etc.). For example, the fluid impermeable barrier 502 includes an outer surface 505, an inner surface (not shown) defining a chamber therein, and an opening 506. The opening 506 allows the porous material 115 disposed at least partially within the chamber to be at least partially exposed to the environment outside of the chamber, such as to receive a fluid (e.g., urine). The fluid collection device 500 includes a reservoir as disclosed herein with respect to the fluid collection device 100 **(****FIG. 1****),** such as in the second end region 527.

The fluid impermeable barrier 502 at least partially defines a first end region 525 and a second end region 527 of the fluid collection device 500. The first end region 525 includes an aperture sized and positioned to receive the conduit 108 therethrough. The second end region 527 of the fluid impermeable barrier 502 may define and include a landing 528 for receiving and holding (*e.g.,* adhering, mechanically retaining) the means for retaining 530 thereon. For example, the landing 528 may include a narrower portion of the fluid collection device 500 (*e.g.,* fluid impermeable barrier 502) than the rest of the fluid collection device 500 (*e.g.,* fluid impermeable barrier 502). The landing 528 may include one or more of fasteners, structures, or adhesives to retain the means for retaining 530 thereon. While shown as a narrower portion of the fluid collection device 500, the landing 528 may have different configurations, such as a flat end surface, or a threaded connection to connect to a corresponding shaped part of the means for retaining 530.

The means for retaining 530 includes an attachment sized and shaped to connect to the fluid impermeable barrier in the second end region 527, such as on the landing 528. For example, the attachment may be tube shaped with an inner dimension sized and shaped to fit on the landing 528. The attachment may be fastened, adhered, or otherwise retained on the landing 528.

The attachment of the means for retaining 530 includes a longitudinally extending member 532. The longitudinally extending member 532 includes a plurality of longitudinally collapsing ring segments connected to each other in series. The longitudinally collapsing ring segments may each collapse or expand independently to adjust an overall length of the fluid collection device 500. Each of the longitudinally collapsing ring segments may at least temporarily lock in an extended or collapsed configuration. For example, the longitudinally collapsing ring segments may be sized and shaped to at least temporarily lock in an extended or collapsed configuration unless subjected to a selected amount of tension or compression, upon which the segments collapse or expand. Accordingly and as shown in **FIG. 5B****,** the longitudinally extending member 532 may be selectively collapsed or extended to a selected length. **FIG. 5B** depicts the longitudinally extending member 532 of the attachment in an extended configuration. The longitudinally extending member 532 may be extended to a selected length to increase the surface area in contact with a wearer in the perineal region and/or gluteal cleft, compared to the collapsed conformation. The means for retaining 530 may provide increased surface contact or a physical anchor on an anatomical feature of the wearer, such as in the gluteal cleft or between the legs.

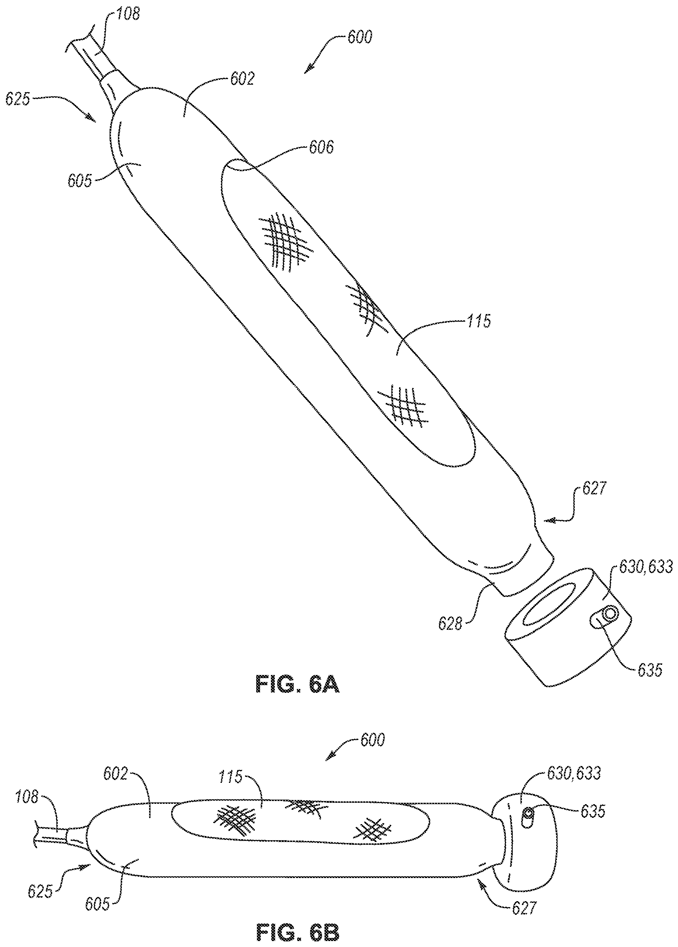

In some examples, the attachment of the means for retaining may differ from the longitudinally extending member 532. **FIG. 6A** is an exploded isometric view of a fluid collection device 600, according to an embodiment. **FIG. 6B** is a side view of the fluid collection device 600 of **FIG. 6A** in a deployed state, according to an embodiment. The fluid collection device 600 includes the porous material 115, the conduit 108, the fluid impermeable barrier 602, and the means for retaining 630. The fluid collection device 600 may be similar or identical to any of the fluid collection devices disclosed herein, in one or more aspects. The fluid impermeable barrier 602 may be similar or identical to the fluid impermeable barrier 102 **(FIGS. 1A-1D),** 302 **(****FIG. 3A****),** 402 **(****FIG. 4A****),** or 502 **(****FIG. 5A-5B****),** in one or more aspects (*e.g*., material composition, position, chamber, etc.). For example, the fluid impermeable barrier 602 includes an outer surface 605, an inner surface (not shown) defining a chamber therein, and an opening 606. The opening 606 allows the porous material 115 disposed at least partially within the chamber to be at least partially exposed to the environment outside of the chamber, such as to receive a fluid (*e.g*., urine). The fluid collection device 600 includes a reservoir as disclosed herein with respect to the fluid collection device 100 **(****FIG. 1****),** such as in the second end region 627.

The fluid impermeable barrier 602 at least partially defines a first end region 625 and a second end region 627 of the fluid collection device 600. The first end region 625 includes an aperture sized and positioned to receive the conduit 108 therethrough. The second end region 627 of the fluid impermeable barrier 602 may define and include a landing 628 for receiving and holding the means for retaining 630. For example, the landing 628 may include a narrower portion of the fluid collection device 600 (*e.g.,* fluid impermeable barrier 602) than the rest of the fluid collection device 600 (*e.g.,* fluid impermeable barrier 602). The landing 628 may include one or more fasteners, structures, or adhesives to retain the means for retaining 630 thereon. While shown as a narrower portion of the fluid collection device 600, the landing 628 may have different configurations, such as a flat end surface, or a threaded connection to connect to a corresponding shaped part of the means for retaining.

The means for retaining 630 includes an attachment sized and shaped to connect to the fluid impermeable barrier in the second end region 627, such as on the landing 628. For example, the attachment may be tube shaped with an inner dimension sized and shaped to fit on the landing 628. The attachment may be fastened, adhered, or otherwise retained on the landing 628. In some examples, the landing 628 may be omitted and the attachment may be sized and shaped to interface with (*e.g*., fit on, fit over, fit against) the fluid impermeable barrier 602 without a landing thereon.

The attachment of the means for retaining 630 includes an inflatable member 633. The inflatable member 633 includes one or more flexible walls defining one or more inflatable portions. The inflatable member 633 includes a fluid inlet 635 for inputting fluid into and releasing fluid from the one or more inflatable portions. The fluid inlet 635 may be resealable. The one or more flexible walls may be made of any impermeable material that holds a gas or liquid, such as any of the fluid impermeable barrier materials disclosed herein (*e.g.*, rubber, latex, polychloroprene, silicone, or a nylon). The outer surface of the inflatable member 633 may have any selected surface texture, such as smooth, rough, or the like. The one or more flexible walls may be sized and shaped to expand one or more of radially or longitudinally when filled with a fluid. For example, the one or more flexible walls may be sized and shaped to inflate to one or more selected dimensions. Accordingly and as shown in **FIG. 6B****,** the inflatable member 633 may be expanded radially to provide a larger surface area and/or structure to interface with the wearer's anatomy and resist dislodgment. **FIG. 6B** depicts the inflatable member 633 of the attachment in an inflated configuration. The inflatable member 633 may be extended radially to increase the surface area in contact with a wearer in the perineal region and/or gluteal cleft, compared to the collapsed conformation. In some examples, the inflatable member 633 may alternatively or additionally be expanded longitudinally by addition of a fluid through the fluid inlet 635. The means for retaining 630 may provide a physical anchor on an anatomical feature of the wearer, such as in the gluteal cleft or between the legs.

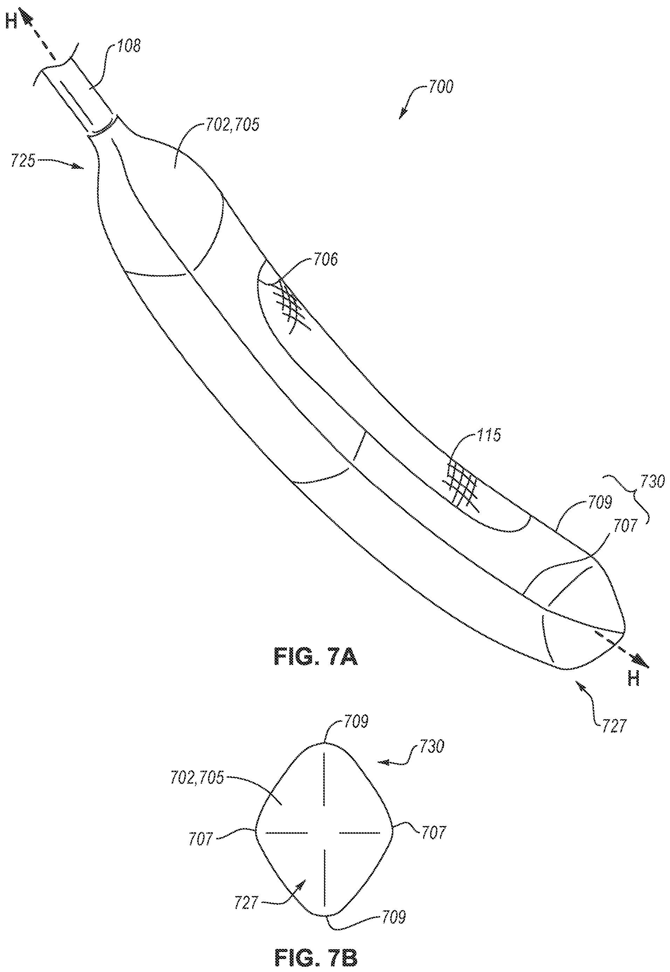

In some examples, the means for retaining may be provided by the overall shape of the fluid collection device. **FIG. 7A** is an isometric view of fluid collection device 700, according to an embodiment. **FIG. 7B** is an end view of the fluid collection device 700 of **FIG. 7A** taken along the longitudinal axis H-H. The fluid collection device 700 includes the porous material 115, the conduit 108, and the fluid impermeable barrier 702 forming the means for retaining 730. The fluid collection device 700 may be similar or identical to any of the fluid collection devices disclosed herein, in one or more aspects. The fluid impermeable barrier 702 may be similar or identical any of the fluid impermeable barriers disclosed herein, in one or more aspects (*e.g.,* material composition, position, chamber, etc.). For example, the fluid impermeable barrier 702 includes an outer surface 705, an inner surface (not shown) defining a chamber therein, and an opening 706. The opening 706 allows the porous material 115 disposed at least partially within the chamber to be at least partially exposed to the environment outside of the chamber, such as to receive a fluid (*e.g.,* urine). The fluid collection device 700 includes a reservoir as disclosed herein with respect to the fluid collection device 100 (**FIG**. **1****),** such as in the second end region 727.

The means for retaining 730 is formed by the shape of the fluid collection device 700 that is at least partially defined by the fluid impermeable barrier 702. The means for retaining 730 includes the fluid impermeable barrier 702 having a rounded prismatic shape. The rounded prismatic shape may include one or more rounded corners 707 and 709 that define the outer shape of the fluid collection device 700. The first rounded corner(s) 707 may at least partially define the lateral shape(s) (*e.g*., sides) of the fluid collection device 700. The second rounded corner(s) 709 may at least partially define the shape(s) of the top and bottom of the fluid collection device 700. As shown, the first rounded corner(s) 707 may exhibit a larger angle than the second rounded corner(s) 709. Accordingly, when viewed along the axis H-H, the rounded prismatic shape of the means for retaining 730 may be generally diamond shaped with rounded corners. The top surface may include the second rounded corner 709 and may have the opening 706 therealong. Accordingly, the porous material 115 may be exposed on the second rounded corner 709. By angling the top surface of the fluid impermeable barrier 702, the fluid collection device 700 may fit more closely to the anatomy of the wearer in the urethral region (*e.g*., perineal region) to resist dislodgement. The rounded corners 707 may also project into the anatomy of the wearer (*e.g.*, legs) to resist dislodgement.

Other shapes may be utilized for fluid collection devices, such as triangular, rounded bottom with an angled top surface, or the like. The fluid impermeable barrier 702 may include an aperture at the first end region 725 or the second end region 727 to allow the conduit 108 to extend into the reservoir in the chamber of the fluid collection device 700.

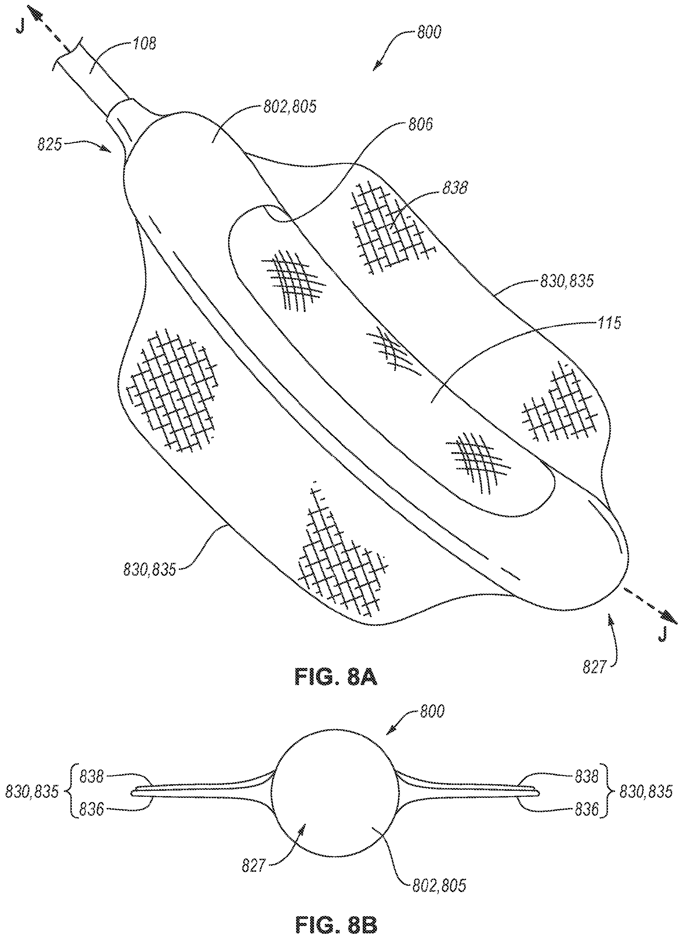

In some examples, the means for retaining may include wings extending from the fluid impermeable barrier. **FIG. 8A** is an isometric view of the fluid collection device 800, according to an embodiment. **FIG. 8B** is an end view of the fluid collection device 800 of **FIG. 8A** viewed along the axis J-J. The fluid collection device 800 includes the porous material 115, the conduit 108, the fluid impermeable barrier 802, and the means for retaining 830. The fluid collection device 800 may be similar or identical to any of the fluid collection devices disclosed herein, in one or more aspects. The fluid impermeable barrier 802 may be similar or identical any of the fluid impermeable barriers disclosed herein, in one or more aspects (*e.g.,* material composition, position, chamber, etc.). For example, the fluid impermeable barrier 802 includes an outer surface 805, an inner surface (not shown) defining a chamber therein, and an opening 806. The opening 806 allows the porous material 115 disposed at least partially within the chamber to be at least partially exposed to the environment outside of the chamber, such as to receive a fluid (e.g., urine). The fluid collection device 800 includes a reservoir as disclosed herein with respect to the fluid collection device 100 **(****FIG. 1****),** such as in the second end region 827.

The means for retaining 830 includes wings 835 extending radially from and longitudinally along the fluid impermeable barrier 802. The wings 835 may include a fluid impermeable material 836. For example, the wings 835 may be at least partially formed of the same material as the fluid impermeable barrier 802, such as integrally formed therewith or attached thereto. The wings 835 may include porous material 838 disposed on the fluid impermeable material 836 (e.g., backing). The porous material 838 may be similar or identical the porous materials disclosed herein, in one or more aspects. For example, the porous material 838 may include any of the fluid permeable membrane materials disclosed herein. The porous material 838 may be disposed on a top or wearer-facing side of the fluid impermeable material 836 of the wing 835. During use, the porous material 838 may provide a softer interface with the wearer than the fluid impermeable material 836 and may also wick fluids, to prevent leakage onto the wearer or the wearers clothes. In some examples, the porous material 838 may be connected to the porous material 115. In such examples, the fluid collected in the porous material 838 may be drawn (e.g., wicked) into the porous material 115.

The wings 835 of the means for retaining 830 may extend longitudinally along substantially all of the fluid impermeable barrier, such as tapering from the first end region 825 to a maximum width and then tapering back to the second end region 827. The wings 835 of the means for retaining 830 may extend longitudinally only a portion of the fluid collection device 800, such as at the first end region 825, the second end region 827, or a medial region therebetween. The wings 835 of the means for retaining 830 may extend longitudinally along at least the opening 806 of the fluid collection device 800.

As shown in **FIG. 8B****,** the wings 835 may extend from the middle (e.g., when viewed from the end) of the fluid impermeable barrier 802. In some examples, the wings 835 may extend from the bottom or the top of the fluid impermeable barrier 802. The wings 835 may extend perpendicularly, tangentially, or obliquely from the outer surface of the fluid impermeable barrier 802.

The wings 835 may extend radially at least 1 cm from the fluid impermeable barrier 802, such as 1 cm to 10 cm, 1 cm to 3 cm, 3 cm to 6 cm, 6 cm to 10 cm, less than 10 cm, less than 6 cm, or less than 4 cm from the fluid impermeable barrier 802. The wing 835 (as a whole) or the components thereof may be at least 1 mm thick, such as 1 mm to 5 mm, 1 mm to 3 mm, 2 mm to 4 mm, 3 mm to 5 mm, less than 5 mm, or less than 3 mm, respectively.

The wings 835 of the means for retaining 830 may be positioned in a wearer's underpants, between the wearer and the underpants, such as in a perineal region thereof. The wings 835 may retain the fluid collection device 800 in position on the wearer by providing larger surface area to engage with the wearer and the wearer's garments (e.g., underpants). The wings 835 may also prevent urine or other fluids from reaching the garments.

In some examples, one or more of any of the means for retaining disclosed herein may be utilized in a single fluid collection device to retain the fluid collection device in position on a wearer. For example, texturing and attachments may be utilized as means for retaining on a single fluid collection device.

Any of the fluid collection devices disclosed herein may be utilized in a fluid collection system. **FIG. 9** is a block diagram of a system 900 for fluid collection, according to an embodiment. The system 900 includes a fluid collection device 901, a fluid storage container 919, and a vacuum source 929. The fluid collection device 901, the fluid storage container 919, and the vacuum source 929 may be fluidly coupled to each other via one or more conduits 108. For example, the fluid collection device 901 may be operably coupled to one or more of the fluid storage container 919 or the (portable) vacuum source 929 via the conduits 108.

The fluid collection device 901 may include any of the fluid collection devices disclosed herein. For example, the fluid collection device 901 may include a fluid impermeable barrier defining an opening, a porous material exposed through the opening, and a means for retaining the fluid collection device in position between the legs of a wearer, as disclosed herein. The fluid collection device 901 may include the conduit 108 including an inlet and an outlet as disclosed herein. The outlet may be fluidly coupled to the fluid storage container 919 and the inlet may be positioned in the fluid collection device 901 such as in a portion of the chamber therein selected to be at a gravimetrically low point of the fluid collection device 901 is worn by a user (e.g., reservoir).

The conduit 108 is coupled to and at least partially extends between one or more of the fluid storage container 919 and the vacuum source 929. In an example, the conduit 108 is directly connected to the vacuum source 929. In such an example, the conduit 108 may extend from the fluid collection device 901 by at least 0,3 m (one foot), at least 0,9 m (three feet), at least 1,5 m (5 feet), or at least 3,0 m (ten feet). In another example, the conduit 108 may be indirectly connected to at least one of the fluid storage container 919 and the vacuum source 929. In some examples, the conduit is secured to a wearer's skin with a catheter securement device, such as a STATLOCK^{®} catheter securement device available from C. R. Bard, Inc., including but not limited to those disclosed in U.S. Patent Nos. 6,117,163; 6,123,398; and 8,211,063.

The inlet (110, **FIG. 1B****)** and the outlet (112, **FIG. 1B****)** may fluidly couple *(e.g.,* directly or indirectly) the vacuum source 929 to the chamber (104 or the reservoir 122 therein, **FIG. 1B****)** of the fluid collection device, such as via one or more connectors thereon. In an example, the inlet and/or the outlet may form a male connector. In another example, the inlet and/or the outlet may form a female connector. In an example, the inlet and/or the outlet may include ribs that are configured to facilitate secure couplings. In an example, the inlet and/or the outlet may form a tapered shape. In an example, the inlet and/or the outlet may include a rigid or flexible material.

Fluid (*e.g.*, urine or other bodily fluids) collected in the fluid collection device 901 may be removed from the chamber thereof via the conduit 108. For example, the first open end of the conduit 108 in the fluid collection device is fluidly connected to the second open end of the conduit 108 which may be disposed in the fluid storage container 919, or the vacuum source 929. The suction force may be introduced into the interior region of the fluid collection device via the first open end of the conduit 108 responsive to a suction (e.g., vacuum) force applied at the second end of the conduit 108. The suction force may be applied to the second open end of the conduit 108 by the vacuum source 929 either directly or indirectly. As the vacuum source 929 applies a vacuum/suction in the conduit 108, the fluid(s) in the chamber *(e.g.,* at the second end region such as in the reservoir 122) of the fluid collection device 901 are drawn into the inlet and out of the fluid collection device via the conduit 108.

The suction force may be applied indirectly via the fluid storage container 919. For example, the second open end *(e.g.,* outlet) of the conduit 108 may be disposed within the fluid storage container 919 and an additional conduit 108 may extend from the fluid storage container 919 to the vacuum source 929. Accordingly, the vacuum source 929 may indirectly apply suction to the fluid collection device via the fluid storage container 919. In such examples, the vacuum source 929 may provide a vacuum/suction through the fluid storage container to the fluid collection device to provide suction in the chamber of the fluid collection device. As the fluid is drained from the chamber of the fluid collection device, the fluid may travel through the first section of conduit 108 to the fluid storage container 919 where it may be retained.

In some examples, the suction force may be applied directly via the vacuum source 929. For example, the second open end of the conduit 108 may be disposed within the vacuum source 929. An additional conduit 108 may extend from the vacuum source 929 to a point outside of the fluid collection device, such as to the fluid storage container 919. In such examples, the vacuum source 929 may be disposed between the fluid collection device and the fluid storage container 919. In examples, the fluid storage container 919 may include a bag (*e.g.,* drainage bag), a bottle or cup *(e.g.,* collection jar), a canister, or any other enclosed container for storing bodily fluids such as urine. In some examples, the fluid storage container 919 may be a rigid container.

The vacuum source 929 may include a wall mounted vacuum line, such as found in hospital rooms. The vacuum source 929 may include one or more of a manual vacuum pump, and electric vacuum pump, a diaphragm pump, a centrifugal pump, a hand pump, a displacement pump, a magnetically driven pump, a peristaltic pump, or any pump configured to produce a vacuum. In examples, the vacuum source 929 may be powered by one or more of a power cord *(e.g.,* connected to a power socket), one or more batteries, or even manual power *(e.g.,* a hand operated vacuum pump). The vacuum sources 929 disclosed herein may include one or more of a switch, a button, a plug, a remote, or any other device suitable to activate the vacuum source 929.

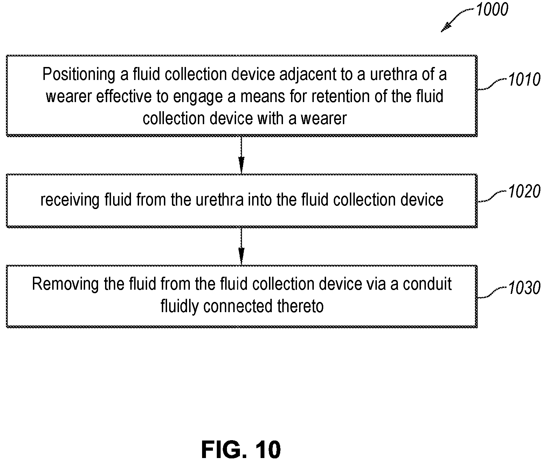

**FIG. 10** is a flow diagram of a method to collect fluid, according to an embodiment. The method 1000 includes block 1010, which recites "positioning a fluid collection device adjacent to a urethra of a wearer effective to engage a means for retaining of the fluid collection device with a wearer." Block 1010 may be followed by Block 1020, which recites "receiving fluid from the urethra into the fluid collection device." Block 1020 may be followed by Block 1030, which recites "removing the fluid from the fluid collection device via a conduit fluidly connected thereto." Blocks 1010, 1020, 1030 of the method 1000 are for illustrative purposes. For example, the block 1010, 1020, 1030 of the method 1000 may be performed in different orders, split into multiple blocks, modified, supplemented, or combined. In an example, one or more of the blocks 1010, 1020, 1030 of the method 1000 may be omitted from the method 1000. For example, the block 1010 of positioning a fluid collection device adjacent to a urethra of a wearer effective to engage a means for retaining of the fluid collection device with a wearer may be omitted.

Block 1010 recites "positioning a fluid collection device adjacent to a urethra of a wearer effective to engage a means for retaining of the fluid collection device with a wearer." The fluid collection device may include any of the fluid collection devices disclosed herein (including any of the means for retaining disclosed herein). For example, the fluid collection device includes a fluid impermeable barrier having an outer surface and an inner surface, the inner surface at least partially defining a chamber, the fluid impermeable barrier also defining an opening extending therethrough, the opening configured to be positioned adjacent to a urethra. The fluid collection device includes a porous material at least partially disposed in the chamber. The fluid impermeable barrier at least partially defines a first end region and a second end region of the fluid collection device, wherein the first end region includes an aperture sized and positioned to receive a conduit (e.g., drainage tube) therethrough. The fluid collection device includes the means for retaining the fluid collection device in a position for use between the legs of a wearer.

The means for retaining aids in retaining the fluid collection device in position during use and may engage or further one or more anatomical features of the wearer. For example, the means for retaining may provide more surface area to contact one or more of the legs, gluteal cleft, perineal region, or the undergarments of the wearer. Accordingly, positioning a fluid collection device adjacent to a urethra of a wearer effective to engage a means for retaining of the fluid collection device with a wearer may include positioning at least a portion of the fluid collection device between the legs of the wearer, in the perineal region of the wearer, or in the gluteal cleft of the wearer. For example, at least a portion of the porous material may be disposed over the urethra of the wearer and another portion may be disposed between the legs or in the gluteal cleft (e.g., intergluteal cleft). The porous material may contact the labia of the wearer.

The means for retaining may include one or more of any of the means for retaining disclosed herein. For example, the means for retaining may include a plurality of flanges in the fluid impermeable barrier, wherein the plurality of flanges are at least partially malleable. The plurality of flanges may include any of the flanges disclosed herein. In such examples, positioning a fluid collection device adjacent to the urethra of a wearer effective to engage a means for retaining of the fluid collection device with a wearer may include placing at least some of the plurality of flanges in contact with the wearer, such as in contact with one or more of the inner legs or buttocks of the wearer. Such positioning may include manipulating or bending the plurality of flanges.

In some examples, the means for retaining may include texturing formed on the outer surface of the fluid impermeable barrier or disposed over the outer surface of the fluid impermeable barrier. The texturing may include any of the texturing disclosed herein. In such examples, positioning a fluid collection device adjacent to the urethra of a wearer effective to engage a means for retaining of the fluid collection device with a wearer may include placing the texturing in contact with the wearer, such as in contact with the skin of the wearer.

In some examples, the means for retaining includes an attachment connected to the fluid impermeable barrier in the second end region. The attachment may include any of the attachments disclosed herein such as the longitudinally extending member or the inflatable member. In such examples, positioning a fluid collection device adjacent to the urethra of a wearer effective to engage a means for retaining of the fluid collection device with a wearer may include placing the attachment in contact with the wearer. Such placement may include extending the longitudinally extending member as selected length or inflating the inflatable member to a selected length and/or outer lateral dimension(s). Positioning a fluid collection device adjacent to the urethra of a wearer effective to engage a means for retaining of the fluid collection device with a wearer may include positioning the attachment between the legs of the wearer and/or in the gluteal cleft of the wearer.

In some examples, the means for retaining includes the fluid impermeable barrier having a rounded prismatic shape having one or more rounded corners. The means for retaining may include any of the shapes of the fluid impermeable barriers disclosed herein. In such examples, positioning a fluid collection device adjacent to the urethra of a wearer effective to engage a means for retaining of the fluid collection device with a wearer may include positioning one or more rounded corners in a cleft between the legs of the wearer, such as one or more of where the thigh meets the perineum, between the labia, or in the intergluteal cleft.

In some examples, the means for retaining includes wings extending radially from and longitudinally along the fluid impermeable barrier, wherein the wings may include porous material. The wings may include any of the wings disclosed herein. In such examples, positioning a fluid collection device adjacent to the urethra of a wearer effective to engage a means for retaining of the fluid collection device with a wearer may include one or more of positioning the wings in contact with skin of the wearer or securing the wings within an undergarment of the wearer. In such examples, the wings may provide additional surface area to provide frictional engagement with the wearer and/or the wearer's undergarment. Positioning a fluid collection device adjacent to the urethra of a wearer effective to engage a means for retaining of the fluid collection device with a wearer may include positioning the porous material of the wings in contact with skin of the wearer.

The block 1020 of receiving fluid from the urethra into the fluid collection device may include receiving the fluid in the porous material. Receiving fluid from the urethra into the fluid collection device may include receiving the fluid in the porous material via the opening. The block 1020 of receiving fluid from the urethra into the fluid collection device may include wicking the fluid(s) from the urethra via the porous material, such as via a fluid permeable membrane and a fluid permeable support. Receiving fluid from the urethra into the fluid collection device may include wicking the fluid into the chamber of the fluid collection device via the porous material. In some examples, the block 1020 may include flowing the fluid towards a portion of the chamber of the fluid collection device that is fluidly coupled to an inlet of a conduit (which may be in fluid communication with a vacuum source). For instance, block 1020 may include flowing the fluid(s) to a substantially unoccupied portion of the chamber (e.g., a reservoir), to a gravimetrically low point of the chamber, etc.

The block 1030 of removing the fluid from the fluid collection device may include applying suction with a vacuum source fluidly connected to the conduit effective to suction the fluid from the chamber via the conduit. Applying suction may include using any of the vacuum sources disclosed herein. The vacuum source may be spaced from the fluid collection device, such as via one or more portions of conduit. In some examples, a fluid storage container is disposed between the vacuum source and the fluid collection device. The block 1030 may include activating the vacuum source (e.g., suction device) in fluid communication with the inlet of the conduit in the fluid collection device. In some examples, activating the vacuum source may include supplying power to the vacuum source by one or more of flipping an on/off switch, pressing a button, plugging the vacuum source into a power outlet, putting batteries into the vacuum source, etc. In some examples, the vacuum source may include a hand operated vacuum pump and applying suction with a vacuum source may include manually operating the hand operated vacuum pump effective to suction the fluid(s) from the chamber via the conduit.

In some examples, applying suction with a vacuum source effective to suction the fluid(s) from the chamber via a conduit disposed therein and fluidly coupled to the vacuum source may be effective to remove at least some fluid (*e.g.,* urine) from the chamber (*e.g.,* interior region) of the fluid collection device. In some examples, applying suction with a vacuum source effective to suction the fluid(s) from the chamber via a conduit disposed therein and fluidly coupled to the vacuum source may be effective to transfer at least some of the fluid from the chamber of the fluid collection device to a fluid storage container (*e.g*._{,} a bottle or bag). In some examples, applying suction with a vacuum source effective to suction the fluid(s) from the chamber may include removing fluid from one or more of a reservoir, fluid permeable support, or fluid permeable membrane of the fluid collection device.

Removing the fluid from the fluid collection device may include allowing the fluid to at least partially drain from the fluid collection device via gravity.

In an example, the method 1000 may include collecting the fluid(s) that are removed from the fluid collection device, such as into the fluid storage container that is spaced from the fluid collection device and fluidly coupled to the conduit. The fluid storage container may include any of the fluid storage containers disclosed herein.

The method 1000 may include forming the fluid collection device such as assembling the means for retaining on the fluid collection device.

The means for retaining of the fluid collection devices, systems, and methods disclosed herein retain the fluid collection devices in position during on the wearer to maintain the spatial relationship between the opening on the fluid collection device with the urethra of the wearer. For example, the means for retaining of the fluid collection devices, systems, and methods disclosed herein provide for secure positioning of the fluid collection device(s) between the legs of wearers that resists dislodgement by external forces. Such resistance may be provided by one or more of friction cause by increased surface contact between the wearer and the fluid collection device, a physical anchor in an anatomical feature, or better anatomical fit with the wearer, all due to the various means for retaining.

While various aspects and embodiments have been disclosed herein, other aspects and embodiments are contemplated. The various aspects and embodiment disclosed herein are for purposes of illustration and are not intended to be limiting.

## Claims

1. A fluid collection device, comprising:
a fluid impermeable barrier (102; 202; 302; 402; 502; 602; 702; 802) having an outer surface (105; 205; 305; 405; 505; 605; 705; 805) and an inner surface (103; 203), the inner surface (103; 203) at least partially defining a chamber (104; 204), the fluid impermeable barrier (102; 202; 302; 402; 502; 602; 702; 802) also defining an opening (106; 206; 306; 406; 506; 606; 706; 806) extending therethrough, the opening (106; 206; 306; 406; 506; 606; 706; 806) configured to be positioned adjacent to a urethra;
a porous material (115; 215) at least partially disposed in the chamber (104; 204); and
a means for retaining the fluid collection device in a position for use between the legs of a wearer,
**characterised in that** the means for retaining includes texturing (336; 436) formed on the outer surface (105; 205; 305; 405; 505; 605; 705; 805) of the fluid impermeable barrier (102; 202; 302; 402; 502; 602; 702; 802) or disposed over the outer surface (105; 205; 305; 405; 505; 605; 705; 805) of the fluid impermeable barrier (102; 202; 302; 402; 502; 602; 702; 802), wherein the texturing (336; 436) includes one or more of:
ridges (437) and valleys (438) on a first portion of the fluid impermeable barrier (102; 202; 302; 402; 502; 602; 702; 802) and a second portion of the fluid impermeable barrier (102; 202; 302; 402; 502; 602; 702; 802) without the texturing (336; 436); or
a three-dimensional pattern sized and shaped to create suction between the texturing (336; 436) and a smooth surface when the smooth surface is applied thereto.

2. The fluid collection device of claim 1 wherein the porous material (115; 215) includes:
a fluid permeable membrane (118) disposed within the chamber (104; 204) and extending across the opening (106; 206; 306: 406; 506; 606; 706; 806); and
a fluid permeable support (120) disposed within the chamber (104; 204) beneath the fluid permeable membrane (118).

3. The fluid collection device of claim 1 or 2, further comprising a conduit (108) including an inlet (110) and an outlet (112), the inlet (110) being positioned within the fluid collection device and the outlet (112) is configured to be fluidly connected to a fluid storage container (919).

4. The fluid collection device of claim 3 wherein:
the fluid impermeable barrier (102; 202; 302; 402; 502; 602; 702; 802) and one or more of the fluid permeable membrane (118) or fluid permeable support (120) define a reservoir (122; 222) therebetween; and
the inlet (110) is disposed in the reservoir (122; 222).

5. The fluid collection device of claim 1, further comprising two sets of longitudinally extending flanges (232, 234) separated by a web (238) therebetween.

6. The fluid collection device of claim 1 wherein the ridges (437) and valleys(438) are sized and shaped to make the first portion of the fluid impermeable barrier (102; 202; 302; 402; 502; 602; 702; 802) having the texturing (336; 436) more compressible than the second portion of the fluid impermeable barrier (102; 202; 302; 402; 502; 602; 702; 802) without the texturing (336; 436).

7. The fluid collection device of claim 1 wherein the three-dimensional pattern includes a repeating diamond pattern with a raised apex in a centroid of at least some diamonds in the repeating diamond pattern.

8. The fluid collection device of claim 1 wherein the smooth surface includes skin.

9. The fluid collection device of claim 1 wherein:
the fluid impermeable barrier (102; 202; 302; 402; 502; 602; 702; 802) at least partially defines a first end region (125; 225; 325; 425; 525; 625; 725; 825) and a second end region (127; 227; 327; 427; 527; 627; 727; 827) of the fluid collection device, wherein the first end region (125; 225; 325; 425; 525; 625; 725; 825) includes an aperture sized and positioned to receive a conduit therethrough; and
the fluid collection device includes an attachment connected to the fluid impermeable barrier (102; 202; 302; 402; 502; 602; 702; 802) in the second end region(127; 227; 327; 427; 527; 627; 727; 827).

10. The fluid collection device of claim 9 wherein the attachment includes a longitudinally extending member (532) having a plurality of longitudinally collapsing ring segments.

11. The fluid collection device of claim 9 wherein the attachment includes an inflatable member (633) sized and shaped to expand one or more of longitudinally or radially.

12. The fluid collection device of claim 1 wherein the fluid impermeable barrier (102; 202; 302; 402; 502; 602; 702; 802) has a rounded prismatic shape and the opening (106; 206; 306; 406; 506; 606; 706; 806) is located on a corner of the rounded prismatic shape.

13. A fluid collection system, comprising:
a fluid storage container (919) configured to hold a fluid;
a fluid collection device according to any of the preceding claims;
a conduit (108) including an inlet (110) and an outlet (112), the outlet (112) being fluidly coupled to the fluid storage container (919) and the inlet (110) being positioned in the chamber (104; 204); and
a vacuum source (929) fluidly coupled to one or more of the fluid storage container (919) or the fluid collection device via the conduit (108), the vacuum source (929) configured to draw fluid from the fluid collection device via the conduit (108).

14. The fluid collection system of claim 13 wherein the vacuum source (929) includes one or more of a vacuum pump, a vacuum line, or a hand pump.

15. The fluid collection system of claim 13 wherein the fluid storage container (919) includes one or more of a canister or a bag.

## Patentansprüche

1. Flüssigkeitssammelvorrichtung, umfassend:
eine flüssigkeitsundurchlässige Barriere (102; 202; 302; 402; 502; 602; 702; 802) mit einer Außenfläche (105; 205; 305; 405; 505; 605; 705; 805) und einer Innenfläche (103; 203), wobei die Innenfläche (103; 203) zumindest teilweise eine Kammer (104; 204) definiert, wobei die flüssigkeitsundurchlässige Barriere (102; 202; 302; 402; 502; 602; 702; 802) auch eine Öffnung (106; 206; 306; 406; 506; 606; 706; 806) definiert, die sich durch sie hindurch erstreckt, wobei die Öffnung (106; 206; 306; 406; 506; 606; 706; 806) konfiguriert ist, um benachbart zu einer Harnröhre positioniert zu sein; ein poröses Material (115; 215), das zumindest teilweise in der Kammer (104; 204) angeordnet ist; und
ein Mittel zum Festhalten der Flüssigkeitssammelvorrichtung in einer Position zur Verwendung zwischen den Beinen eines Trägers,
**dadurch gekennzeichnet, dass** das Mittel zum Festhalten eine Texturierung (336; 436) enthält, die an der Außenfläche (105; 205; 305; 405; 505; 605; 705; 805) der flüssigkeitsundurchlässigen Barriere (102; 202; 302; 402; 502; 602; 702; 802) ausgebildet oder über der Außenfläche (105; 205; 305; 405; 505; 605; 705; 805) der flüssigkeitsundurchlässigen Barriere (102; 202; 302; 402; 502; 602; 702; 802) angeordnet ist, wobei die Texturierung (336; 436) eines oder mehrere der Folgenden enthält:
Rippen (437) und Täler (438) auf einem ersten Abschnitt der flüssigkeitsundurchlässigen Barriere (102; 202; 302; 402; 502; 602; 702; 802) und einem zweiten Abschnitt der flüssigkeitsundurchlässigen Barriere (102; 202; 302; 402; 502; 602; 702; 802) ohne die Texturierung (336; 436); oder
ein dreidimensionales Muster, das bemessen und geformt ist, um einen Sog zwischen der Texturierung (336; 436) und einer glatten Oberfläche zu erzeugen, wenn die glatte Oberfläche darauf aufgebracht wird.

2. Flüssigkeitssammelvorrichtung nach Anspruch 1, wobei das poröse Material (115; 215) Folgendes enthält:
eine flüssigkeitsdurchlässige Membran (118), die innerhalb der Kammer (104; 204) angeordnet ist und sich über die Öffnung (106; 206; 306; 406; 506; 606; 706; 806) erstreckt; und
eine flüssigkeitsdurchlässige Stütze (120), die innerhalb der Kammer (104; 204) unter der flüssigkeitsdurchlässigen Membran (118) angeordnet ist.

3. Flüssigkeitssammelvorrichtung nach Anspruch 1 oder 2, ferner umfassend eine Leitung (108), die einen Einlass (110) und einen Auslass (112) beinhaltet, wobei der Einlass (110) innerhalb der Flüssigkeitssammelvorrichtung positioniert ist und der Auslass (112) konfiguriert ist, um fluidisch mit einem Flüssigkeitsspeicherbehälter (919) verbunden zu sein.

4. Flüssigkeitssammelvorrichtung nach Anspruch 3, wobei:
die flüssigkeitsundurchlässige Barriere (102; 202; 302; 402; 502; 602; 702; 802) und eine oder mehrere der flüssigkeitsdurchlässigen Membran (118) oder der flüssigkeitsdurchlässigen Stütze (120) dazwischen ein Reservoir (122; 222) definieren; und
der Einlass (110) in dem Reservoir (122; 222) angeordnet ist.

5. Flüssigkeitssammelvorrichtung nach Anspruch 1, ferner umfassend zwei Sätze von sich in Längsrichtung erstreckenden Flanschen (232, 234), die durch einen Steg (238) dazwischen getrennt sind.

6. Flüssigkeitssammelvorrichtung nach Anspruch 1, wobei die Rippen (437) und Täler (438) so bemessen und geformt sind, dass der erste Abschnitt der flüssigkeitsundurchlässigen Barriere (102; 202; 302; 402; 502; 602; 702; 802) mit der Texturierung (336; 436) komprimierbarer ist als der zweite Abschnitt der flüssigkeitsundurchlässigen Barriere (102; 202; 302; 402; 502; 602; 702; 802) ohne die Texturierung (336; 436).

7. Flüssigkeitssammelvorrichtung nach Anspruch 1, wobei das dreidimensionale Muster ein sich wiederholendes Rautenmuster mit einem erhöhten Scheitelpunkt in einem Schwerpunkt von mindestens einigen Rauten in dem sich wiederholenden Rautenmuster beinhaltet.

8. Flüssigkeitssammelvorrichtung nach Anspruch 1, wobei die glatte Oberfläche Haut enthält.

9. Flüssigkeitssammelvorrichtung nach Anspruch 1, wobei:
die flüssigkeitsundurchlässige Barriere (102; 202; 302; 402; 502; 602; 702; 802) zumindest teilweise einen ersten Endbereich (125; 225; 325; 425; 525; 625; 725; 825) und einen zweiten Endbereich (127; 227; 327; 427; 527; 627; 727; 827) der Flüssigkeitssammelvorrichtung definiert, wobei der erste Endbereich (125; 225; 325; 425; 525; 625; 725; 825) eine Öffnung enthält, die so bemessen und positioniert ist, dass sie eine Leitung durch sie hindurch aufnimmt; und
die Flüssigkeitssammelvorrichtung einen Aufsatz beinhaltet, der mit der flüssigkeitsundurchlässigen Barriere (102; 202; 302; 402; 502; 602; 702; 802) in dem zweiten Endbereich (127; 227; 327; 427; 527; 627; 727; 827) verbunden ist.

10. Flüssigkeitssammelvorrichtung nach Anspruch 9, wobei der Aufsatz ein sich in Längsrichtung erstreckendes Element (532) beinhaltet, das eine Vielzahl von in Längsrichtung kollabierenden Ringsegmenten aufweist.

11. Flüssigkeitssammelvorrichtung nach Anspruch 9, wobei der Aufsatz ein aufblasbares Element (633) beinhaltet, das so bemessen und geformt ist, dass es sich in Längsrichtung oder radial ausdehnt.

12. Flüssigkeitssammelvorrichtung nach Anspruch 1, wobei die flüssigkeitsundurchlässige Barriere (102; 202; 302; 402; 502; 602; 702; 802) eine abgerundete prismatische Form aufweist und sich die Öffnung (106; 206; 306; 406; 506; 606; 706; 806) an einer Ecke der abgerundeten prismatischen Form befindet.

13. Flüssigkeitssammelsystem, umfassend:
einen Flüssigkeitsspeicherbehälter (919), der zur Aufnahme einer Flüssigkeit konfiguriert ist;
eine Flüssigkeitssammelvorrichtung nach einem der vorhergehenden Ansprüche;
eine Leitung (108), die einen Einlass (110) und einen Auslass (112) beinhaltet, wobei der Auslass (112) fluidisch mit dem Flüssigkeitsspeicherbehälter (919) gekoppelt ist und der Einlass (110) in der Kammer (104; 204) positioniert ist; und
eine Vakuumquelle (929), die über die Leitung (108) fluidisch mit einem oder mehreren von dem Flüssigkeitsspeicherbehälter (919) oder der Flüssigkeitssammelvorrichtung gekoppelt ist, wobei die Vakuumquelle (929) dazu konfiguriert ist, Flüssigkeit aus der Flüssigkeitssammelvorrichtung über die Leitung (108) abzuziehen.

14. Flüssigkeitssammelsystem nach Anspruch 13, wobei die Vakuumquelle (929) eine oder mehrere von einer Vakuumpumpe, einer Vakuumlinie oder einer Handpumpe beinhaltet.

15. Flüssigkeitssammelsystem nach Anspruch 13, wobei der Flüssigkeitsspeicherbehälter (919) eines oder mehrere von einem Kanister oder einem Beutel beinhaltet.

## Revendications

1. Dispositif de collecte des fluides comprenant :
une barrière imperméable aux fluides (102 ; 202 ; 302 ; 402 ; 502 ; 602 ; 702 ; 802) ayant une surface extérieure (105 ; 205 ; 305 ; 405 ; 505 ; 605 ; 705 ; 805) et une surface intérieure (103 ; 203), la surface intérieure (103 ; 203) définissant au moins partiellement une chambre (104 ; 204), la barrière imperméable aux fluides (102 ; 202 ; 302 ; 402 ; 502 ; 602 ; 702 ; 802) définissant également une ouverture (106 ; 206 ; 306 ; 406 ; 506 ; 606 ; 706 ; 806) s'étendant à travers elle, l'ouverture (106 ; 206 ; 306 ; 406 ; 506 ; 606 ; 706 ; 806) configurée pour être positionnée à proximité d'un urètre ;
un matériau poreux (115 ; 215) disposé au moins partiellement dans la chambre (104 ; 204) ; et
un moyen de maintenir le dispositif de collecte des fluides dans une position permettant de l'utiliser entre les jambes d'un porteur,
**caractérisé en ce que** le moyen de rétention comprend une texturation (336 ; 436) formée sur la surface extérieure (105 ; 205 ; 305 ; 405 ; 505 ; 605 ; 705 ; 805) de la barrière imperméable aux fluides (102 ; 202 ; 302 ; 402 ; 502 ; 602 ; 702 ; 802) ou disposée sur la surface extérieure (105 ; 205 ; 305 ; 405 ; 505 ; 605 ; 705 ; 805) de la barrière imperméable aux fluides (102 ; 202 ; 302 ; 402 ; 502 ; 602 ; 702 ; 802), dans laquelle la texturation (336 ; 436) comprend un ou plusieurs des éléments suivants :
des crêtes (437) et des vallées (438) sur une première partie de la barrière imperméable aux fluides (102 ; 202 ; 302 ; 402 ; 502 ; 602 ; 702 ; 802) et une seconde partie de la barrière imperméable aux fluides (102 ; 202 ; 302 ; 402 ; 502 ; 602 ; 702 ; 802) sans la texturation (336 ; 436) ; ou
un motif tridimensionnel dimensionné et façonné pour créer une succion entre la texturation (336 ; 436) et une surface lisse lorsque la surface lisse est appliquée sur cette dernière.

2. Dispositif de collecte des fluides selon la revendication 1 dans lequel le matériau poreux (115 ; 215) comprend :
une membrane perméable aux fluides (118) disposée dans la chambre (104 ; 204) et s'étendant à travers l'ouverture (106 ; 206 ; 306 ; 406 ; 506 ; 606 ; 706 ; 806) ; et
un support perméable aux fluides (120) disposé dans la chambre (104 ; 204) sous la membrane perméable aux fluides (118).

3. Dispositif de collecte des fluides selon la revendication 1 ou 2, comprenant en outre un conduit (108) comprenant une entrée (110) et une sortie (112), l'entrée (110) étant positionnée à l'intérieur du dispositif de collecte des fluides et la sortie (112) étant configurée pour être connectée fluidiquement à un récipient de stockage des fluides (919).

4. Dispositif de collecte des fluides selon la revendication 3, dans lequel :
la barrière imperméable aux fluides (102 ; 202 ; 302 ; 402 ; 502 ; 602 ; 702 ; 802) et une ou plusieurs membranes perméables aux fluides (118) ou un support perméable aux fluides (120) définissent un réservoir (122 ; 222) entre eux ; et
l'entrée (110) est disposée dans le réservoir (122 ; 222).

5. Dispositif de collecte des fluides selon la revendication 1, comprenant en outre deux ensembles de brides s'étendant longitudinalement (232, 234) séparées par une bande (238) entre elles.

6. Dispositif de collecte des fluides selon la revendication 1 dans lequel les crêtes (437) et les vallées (438) sont dimensionnées et formées pour rendre la première partie de la barrière imperméable aux fluides (102 ; 202 ; 302 ; 402 ; 502 ; 602 ; 702 ; 802) ayant la texturation (336 ; 436) plus compressible que la seconde partie de la barrière imperméable aux fluides (102 ; 202 ; 302 ; 402 ; 502 ; 602 ; 702 ; 802) sans la texturation (336 ; 436).

7. Dispositif de collecte des fluides selon la revendication 1, dans lequel le motif tridimensionnel comprend un motif de diamant répétitif avec un sommet surélevé dans un centroïde d'au moins quelques diamants dans le motif de diamant répétitif.

8. Dispositif de collecte des fluides selon la revendication 1, dans lequel la surface lisse comprend de la peau.

9. Dispositif de collecte des fluides selon la revendication 1, dans lequel :
la barrière imperméable aux fluides (102 ; 202 ; 302 ; 402 ; 502 ; 602 ; 702 ; 802) définit au moins partiellement une première zone d'extrémité (125 ; 225 ; 325 ; 425 ; 525 ; 625 ; 725 ; 825) et une seconde zone d'extrémité (127 ; 227 ; 327 ; 427 ; 527 ; 627 ; 727 ; 827) du dispositif de collecte des fluides, dans lequel la première région d'extrémité (125 ; 225 ; 325 ; 425 ; 525 ; 625 ; 725 ; 825) comprend une ouverture dimensionnée et positionnée pour recevoir un conduit à travers elle ; et
le dispositif de collecte des fluides comprend une attache reliée à la barrière imperméable aux fluides (102 ; 202 ; 302 ; 402 ; 502 ; 602 ; 702 ; 802) dans la seconde région d'extrémité (127 ; 227 ; 327 ; 427 ; 527 ; 627 ; 727 ; 827).

10. Dispositif de collecte des fluides selon la revendication 9, dans lequel l'attache comprend un élément (532) s'étendant longitudinalement et comportant une pluralité de segments d'anneau s'effondrant longitudinalement.

11. Dispositif de collecte des fluides selon la revendication 9, dans lequel l'attache comprend un élément gonflable (633) dimensionné et formé pour se dilater longitudinalement ou radialement.

12. Dispositif de collecte des fluides selon la revendication 1 dans lequel la barrière imperméable aux fluides (102 ; 202 ; 302 ; 402 ; 502 ; 602 ; 702 ; 802) a une forme prismatique arrondie et l'ouverture (106 ; 206 ; 306 ; 406 ; 506 ; 606 ; 706 ; 806) est située sur un coin de la forme prismatique arrondie.

13. Système de collecte des fluides comprenant :
un récipient de stockage des fluides (919) configuré pour contenir un fluide ;
un dispositif de collecte des fluides selon l'une quelconque des revendications précédentes ;
un conduit (108) comprenant une entrée (110) et une sortie (112), la sortie (112) étant couplée fluidiquement au récipient de stockage des fluides (919) et l'entrée (110) étant positionnée dans la chambre (104 ; 204) ; et
une source de vide (929) couplée fluidiquement à un ou plusieurs des récipients de stockage des fluides (919) ou au dispositif de collecte des fluides par l'intermédiaire du conduit (108), la source de vide (929) configurée pour aspirer le fluide du dispositif de collecte des fluides par l'intermédiaire du conduit (108).

14. Système de collecte des fluides selon la revendication 13, dans lequel la source de vide (929) comprend une ou plusieurs pompes à vide, une conduite à vide ou une pompe à main.

15. Système de collecte des fluides selon la revendication 13 dans lequel le récipient de stockage des fluides (919) comprend un ou plusieurs parmi un bidon ou un sac.
